# EUROPEAN PATENT APPLICATION

(11) **EP 4 327 718 A1**
(43) Date of publication of application: **28.02.2024**
(21) Application number: 23193276.5
(22) Date of filing: 24.08.2023
(51) Int. Cl.: A61B 1/00, A61B 1/227, A61B 1/233, A61B 1/267, A61B 1/273

(54) **IMAGE PROCESSING DEVICE, METHOD FOR OPERATING THE SAME, AND ENDOSCOPE SYSTEM**

(30) Priority: 26.08.2022 JP 2022135036
(71) Applicant: FUJIFILM Corporation, Tokyo 106-8620 (JP); Fujita Academy, Toyoake-shi, Aichi 470-1192 (JP)
(72) Inventor: TERAMURA, Yuichi, Kanagawa (JP); OTAKA, Yohei, Toyoake (JP); SHIBATA, Seiko, Toyoake (JP)
(74) Representative: Hughes, Andrea Michelle

(57) **Abstract**

Provided are an image processing device, a method for operating the same, and an endoscope system capable of outputting information for observation support according to an observation target included in a medical image.

A processor (23) included in the image processing device acquires a medical image (31, 32, 33, 72), outputs observation target identification information (41) indicating an observation support target part or indicating that the medical image is out of an observation support target by inputting the medical image to an observation target identification algorithm, selects, from a plurality of observation support algorithms, one specific observation support algorithm based on the observation target identification information, outputs observation support information by inputting the medical image to the specific observation support algorithm, and performs a control of notifying of the observation support information.

## Description

### BACKGROUND OF THE INVENTION

### 1. Field of the Invention

The present invention relates to an image processing device that supports observation, a method for operating the same, and an endoscope system.

### 2. Description of the Related Art

JP2021-48927A discloses an endoscope image processing system that performs a guide display of an observation point, and discloses that "in a case in which the image region B10 designated by the region designation data acquired by the AI processing module 22 corresponds to the observation point, the output processing module 24 performs a guide display as follows, that is, the output processing module 24 superimposes and displays the aiming displays F 1, F2, and F3 on the endoscope video, sets the endoscope image as a still image G4 at a timing at which the image region B 10 becomes a predetermined size within the frame of the aiming display F2 shown in a predetermined position, and produces a performance to frame out the still image G4 while rotating and reducing the still image G4".

In addition, JP2021-61911A discloses an operation support device that generates and displays an action to be executed next as operation support information, and discloses that "the operation support device 20 can display the operation support information by disposing an indicator on the endoscope image".

### SUMMARY OF THE INVENTION

In endoscopy, a structure of an observation target reflected in a medical image may differ greatly depending on an observation site. Therefore, in a case in which endoscopy is performed over a plurality of parts with greatly different internal structures, it may be insufficient to output information for observation support suitable for each observation site in a case in which there is one type of trained model that outputs operation support information, such as position information of an endoscope distal end part and a route along which the endoscope advances, in response to an input of the medical image.

In addition, in the endoscopy, there is a scene in which a medical image that is unsuitable for observation for the examination is acquired. It is desirable to provide a notification to a user who is an operator not only in a scene in which a medical image is acquired in normal observation but also in a scene in which a medical image unsuitable for such observation is acquired.

An object of the present invention is to provide an image processing device, a method for operating the same, and an endoscope system capable of outputting information for observation support according to an observation target included in a medical image.

An image processing device according to an aspect of the present invention comprises: a processor, in which the processor acquires a medical image, outputs observation target identification information indicating an observation support target part included in the medical image or indicating that the medical image is out of an observation support target by inputting the medical image to an observation target identification algorithm, selects, from a plurality of observation support algorithms, one specific observation support algorithm based on the observation target identification information, outputs observation support information by inputting the medical image to the specific observation support algorithm, and performs a control of notifying of the observation support information.

It is preferable that the observation support target part is a nasal cavity, a nasopharynx, an oropharynx, a hypopharynx, a larynx, a trachea, or an esophagus.

It is preferable that the notification of the observation support information is performed by a guide image for displaying the observation support information and/or a voice for notifying of the observation support information.

It is preferable that the observation support information is endoscope position determination information indicating whether a position of an endoscope is appropriate or inappropriate, endoscope operation information indicating an operation method of the endoscope, subject position information for prompting change or confirmation of a position of a subject, and/or observation support stop information indicating that an observation support is stopped.

It is preferable that the endoscope operation information is a moving direction and/or a moving amount of a distal end part of the endoscope, and the moving direction is a right direction, a left direction, an upward direction, a downward direction, a backward direction, a forward direction, a right turn, or a left turn.

It is preferable that the processor performs a control of displaying an endoscope operation support diagram on the guide image as the endoscope operation information.

It is preferable that the processor performs a control of switching a display of the endoscope operation information based on the endoscope position determination information.

It is preferable that the specific observation support algorithm includes an operation support algorithm, and the processor outputs the endoscope operation information or the observation support stop information by inputting the medical image to the operation support algorithm.

It is preferable that the processor inputs a latest medical image to the operation support algorithm.

It is preferable that the operation support algorithm is a trained model that outputs the endoscope operation information and/or the subject position information.

It is preferable that the specific observation support algorithm includes a position determination algorithm and an operation support algorithm, the position determination algorithm is a trained model that outputs the endoscope position determination information in response to an input of the medical image, and the operation support algorithm is a trained model that outputs the endoscope operation information and/or the subject position information in response to an input of the medical image.

It is preferable that the processor inputs a latest medical image to each of the position determination algorithm and the operation support algorithm.

It is preferable that the observation target identification algorithm is a trained model that has been trained using a learning image including the medical image in which a nasal cavity, a nasopharynx, an oropharynx, a hypopharynx, a larynx, a trachea, or an esophagus is included in an observation target.

It is preferable that the processor performs a control of switching between presence and absence of the notification of the observation support information.

It is preferable that, in a case in which the observation support target part output by the observation target identification algorithm is a nasal cavity, the processor outputs insertion region information indicating an upper route insertion region and/or a lower route insertion region, which is a region suitable for insertion of an endoscope, included in the medical image by inputting the medical image to an operation support algorithm, calculates insertion route information that is an area, a width, and/or coordinate information of a center position of the upper route insertion region and/or the lower route insertion region based on the insertion region information, and performs a control of displaying the insertion route information on the guide image as the observation support information.

It is preferable that the processor performs a control of displaying the upper route insertion region or the lower route insertion region on the guide image by changing a display mode thereof based on the insertion route information.

It is preferable that, in a case in which the observation support target part output by the observation target identification algorithm is a nasal cavity, the processor outputs insertion region information indicating an upper route insertion region and/or a lower route insertion region, which is a region suitable for insertion of the endoscope, included in the medical image by inputting the medical image to the operation support algorithm, calculates insertion route information that is an area, a width, and/or coordinate information of a center position of the upper route insertion region and/or the lower route insertion region based on the insertion region information, and outputs the endoscope operation information using the insertion route information.

It is preferable that, in a case in which the observation support target part output by the observation target identification algorithm is a nasopharynx, the processor outputs nasopharyngeal position information indicating that the position of the endoscope is in an appropriate position or in an inappropriate direction position that is an inappropriate right position, an inappropriate left position, an inappropriate upper position, an inappropriate lower position, an inappropriate back position, or an inappropriate front position by inputting the medical image to the operation support algorithm, and outputs the endoscope operation information based on the nasopharyngeal position information.

It is preferable that the operation support algorithm is a trained model that has been trained using a learning image including the medical image associated with the nasopharyngeal position information.

It is preferable that, in a case in which the observation support target part output by the observation target identification algorithm is an oropharynx, and the position determination algorithm to which the medical image is input outputs that the position of the endoscope is inappropriate as the endoscope position determination information, the processor outputs oropharyngeal region information indicating a glottis region and/or an epiglottis region included in the medical image by inputting the medical image to the operation support algorithm, calculates oropharyngeal region arithmetic information that is an area, a width, and/or coordinate information of a center position of the glottis region and the epiglottis region based on the oropharyngeal region information, and outputs the endoscope operation information using the oropharyngeal region arithmetic information.

It is preferable that the position determination algorithm is a trained model that has been trained using a learning image in which the medical image and the endoscope position determination information are associated with each other.

It is preferable that, in a case in which the observation support target part output by the observation target identification algorithm is a hypopharynx, and the position determination algorithm to which the medical image is input outputs that the position of the endoscope is inappropriate as the endoscope position determination information, the processor outputs hypopharyngeal region information indicating a glottis region and/or a vocal fold region included in the medical image by inputting the medical image to the operation support algorithm, calculates hypopharyngeal region arithmetic information that is an area, a width, and/or coordinate information of a center position of the glottis region, and that is a length of the vocal fold region based on the hypopharyngeal region information, and outputs the endoscope operation information using the hypopharyngeal region arithmetic information.

It is preferable that the position determination algorithm is a trained model that has been trained using a learning image in which the medical image and the endoscope position determination information are associated with each other.

It is preferable that, in a case in which the observation support target part output by the observation target identification algorithm is an esophagus or a trachea, the processor outputs the endoscope operation information for providing an instruction to pull out the endoscope by inputting the medical image to the operation support algorithm.

It is preferable that, in a case in which the observation target identification algorithm outputs that the medical image is out of the observation support target as the observation target identification information, the processor outputs the observation support stop information by inputting the medical image to the operation support algorithm.

It is preferable that the observation target identification algorithm is a trained model that has been trained to, in a case in which the medical image including a foreign substance, which is food or saliva, shake, blurriness, or halation is input, output that the medical image is out of the observation support target as the observation target identification information.

A method for operating an image processing device according to an aspect of the present invention comprises: a step of acquiring a medical image; a step of outputting observation target identification information indicating an observation support target part included in the medical image or indicating that the medical image is out of an observation support target by inputting the medical image to an observation target identification algorithm; a step of selecting, from a plurality of observation support algorithms, one specific observation support algorithm based on the observation target identification information; a step of outputting observation support information by inputting the medical image to the specific observation support algorithm; and a step of performing a control of notifying of the observation support information.

An endoscope system according to an aspect of the present invention comprises: the image processing device according to the aspect of the present invention; a light source device that emits illumination light; and an endoscope that images the medical image.

According to the present invention, it is possible to output information for observation support according to an observation target included in a medical image.

### BRIEF DESCRIPTION OF THE DRAWINGS

Fig. 1 is a block diagram showing a function of an image processing device.
Fig. 2 is a schematic view of an endoscope system.
Fig. 3 is a block diagram showing a function of an observation support information generation unit.
Fig. 4 is an explanatory diagram showing an output of observation support information in response to an input of the latest medical image.
Fig. 5 is an image diagram showing an example of a guide image showing endoscope position determination information of "appropriate" in a case in which an oropharyngeal image is input.
Fig. 6 is an image diagram showing an example of a guide image showing endoscope position determination information of "inappropriate" in a case in which the oropharyngeal image is input.
Fig. 7 is an image diagram showing an example of a guide image showing observation support stop information.
Fig. 8 is an explanatory diagram showing swallowing.
Fig. 9 is an explanatory diagram showing an appropriate observation position.
Fig. 10 is an explanatory diagram showing a case in which an endoscope is erroneously inserted into an esophagus.
Fig. 11 is an explanatory diagram showing a case in which the endoscope is erroneously inserted into a trachea.
Fig. 12 is an explanatory diagram showing an insertion route of an endoscope as viewed from a side surface of a subject.
Fig. 13 is an explanatory diagram showing the insertion route of the endoscope as viewed from a front surface of the subject.
Fig. 14 is an image diagram showing an example of an upper route image.
Fig. 15 is an image diagram showing an example of a lower route image.
Fig. 16 is an image diagram showing an example of an anterior nasal image.
Fig. 17 is an image diagram showing an example of an upper route insertion region.
Fig. 18 is an image diagram showing an example of a guide image in which an insertion route guide mark and an image center guide mark are displayed in a case in which the upper route image is input.
Fig. 19 is an image diagram showing an example of a guide image in which an operation instruction in an upward direction is provided using a guide arrow in a case in which the upper route image is input.
Fig. 20 is an image diagram showing an example of a guide image in which the operation instruction in the upward direction is provided using an icon in a case in which the upper route image is input.
Fig. 21 is an image diagram showing an example of a guide image in which an operation instruction in a lower left direction is provided using an icon in a case in which the upper route image is input.
Fig. 22 is an image diagram showing an example of a guide image in which an operation instruction in the lower left direction is provided using an endoscope operation support diagram in a case in which the upper route image is input.
Fig. 23 is an image diagram showing an example of a guide image in which a left turning operation instruction is provided using an endoscope operation support diagram in a case in which the upper route image is input.
Fig. 24 is an image diagram showing an example of a lower route insertion region.
Fig. 25 is an image diagram showing an example in which the upper route insertion region and the lower route insertion region are detected.
Fig. 26 is an image diagram showing an example of a guide image in which an insertion route guide mark and an image center guide mark are displayed in a case in which the upper route insertion region and the lower route insertion region are detected.
Fig. 27 is an image diagram showing an example of a guide image in which an operation instruction is provided in a case in which the upper route insertion region and the lower route insertion region are detected.
Fig. 28 is an explanatory diagram showing heights of a nasopharynx, oropharynx, and hypopharynx.
Fig. 29 is an image diagram showing an example of a nasopharyngeal image in an appropriate observation position.
Fig. 30 is an image diagram showing an example of a guide image in a case in which the nasopharyngeal image in the appropriate observation position is input.
Fig. 31 is an image diagram showing an example of a nasopharyngeal image in an inappropriate observation position.
Fig. 32 is an image diagram showing an example of a guide image in which an operation instruction in a forward direction is provided using an icon in a case in which the nasopharyngeal image is input.
Fig. 33 is an image diagram showing an example of a guide image in which the operation instruction in the forward direction is provided using an endoscope operation support diagram in a case in which the nasopharyngeal image is input.
Fig. 34 is an image diagram showing an example of an oropharyngeal image in an appropriate observation position.
Fig. 35 is an image diagram showing an example of an oropharyngeal image in an inappropriate observation position.
Fig. 36 is an image diagram showing an example of a guide image in a case in which the oropharyngeal image in the appropriate observation position is input.
Fig. 37 is an image diagram showing an example of an oropharyngeal image in which a glottis region and an epiglottis region are detected.
Fig. 38 is an image diagram showing an example of a guide image in which an operation instruction in a forward direction is provided using an icon in a case in which the oropharyngeal image is input.
Fig. 39 is an image diagram showing an example of a guide image showing subject position information in a case in which the oropharyngeal image is input.
Fig. 40 is an image diagram showing an example of a hypopharyngeal image in an appropriate observation position.
Fig. 41 is an image diagram showing an example of a hypopharyngeal image in an inappropriate observation position.
Fig. 42 is an image diagram showing an example of a guide image in a case in which the hypopharyngeal image in the appropriate observation position is input.
Fig. 43 is an image diagram showing an example of a hypopharyngeal image in which a glottis region and a vocal fold region are detected.
Fig. 44 is an image diagram showing an example of a guide image in which an operation instruction in a forward direction is provided using an icon in a case in which the hypopharyngeal image is input.
Fig. 45 is an image diagram showing an example of an esophageal image.
Fig. 46 is an image diagram showing an example of a tracheal image.
Fig. 47 is an image diagram showing an example of a guide image in which an operation instruction in a forward direction is provided using an icon in a case in which the esophageal image is input.
Fig. 48 is an image diagram showing an example of a guide image for displaying a warning.
Fig. 49 is a flowchart showing a method for operating the image processing device.

### DESCRIPTION OF THE PREFERRED EMBODIMENTS

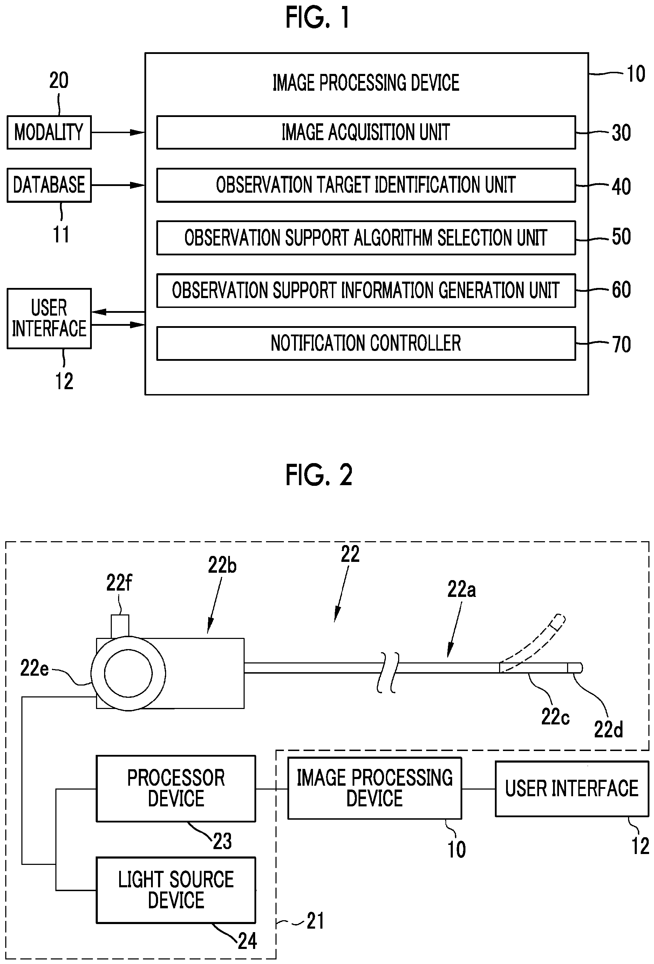

As shown in Fig. 1, an image processing device 10 receives a medical image from a modality 20 that captures the medical image and transmits the medical image to the image processing device 10 or a database 11 in which the medical image is stored. The image processing device 10, the modality 20, and the database 11 are connected to each other so as to be communicable with each other by wire or wirelessly via a network. The network is the Internet, a local area network (LAN), or the like.

The image processing device 10 is connected to a user interface 12 by wire or wirelessly. The user interface 12 is an input device that receives input operations of function settings from a user, such as a keyboard, a mouse, a microphone, a foot switch, a touch pad, a tablet, and a touch pen. In addition, the user interface 12 is an output device such as a display, a head-mounted display, or a speaker that receives a notification instruction from the image processing device 10 and provides the notification to the user. The user interface 12 may be connected to both the image processing device 10 and a processor device 23 described below. That is, the user interface 12 is an input/output device having a function of receiving an input instruction to the image processing device 10 or the processor device 23 and a function of providing an output instruction from the image processing device 10 or the processor device 23.

As shown in Fig. 1, the image processing device 10 includes an image acquisition unit 30, an observation target identification unit 40, an observation support algorithm selection unit 50, an observation support information generation unit 60, and a notification controller 70. The image processing device 10 receives the medical image, performs image processing on the medical image in substantially real time, outputs observation support information, and transmits the information to the user interface 12. As will be described in detail below, the image processing device 10 identifies a part of an observation target included in the medical image or identifies that the medical image is out of an observation support target to output observation target identification information, selects an observation support algorithm according to the observation target identification information, inputs the medical image to the observation support algorithm and outputs the observation support information, and performs a control of notifying the user such as a doctor of the observation support information via the user interface 12.

The image processing device 10 is a computer comprising a processor. A controller (not shown) configured by the processor operates programs related to various kinds of processing or controls incorporated in a program storage memory (not shown) provided in the image processing device 10, thereby realizing functions of the image acquisition unit 30, the observation target identification unit 40, the observation support algorithm selection unit 50, the observation support information generation unit 60, and the notification controller 70 in the image processing device 10.

In the present embodiment, the image processing device 10 is suitable for an application in which the modality 20 is an endoscope system 21 and image processing of a medical image which is an endoscope image is performed. The endoscope system 21 obtains an endoscope image by inserting an endoscope into a living body of a subject by the user and imaging an observation target that can be observed by being illuminated with illumination light. The captured endoscope image is transmitted to the image processing device 10. The user is a doctor who operates the endoscope and is also called an operator.

As shown in Fig. 2, the endoscope system 21 includes an endoscope 22, a processor device 23, and a light source device 24. The endoscope 22 is optically connected to the light source device 24 and electrically connected to the processor device 23. These connections are not limited to wired and may be wireless. The endoscope system 21 may comprise the image processing device 10 in addition to the endoscope 22, the processor device 23, and the light source device 24.

The endoscope 22 includes an insertion part 22a to be inserted into a body of the subject, an operating part 22b provided at a base end portion of the insertion part 22a, and a bendable part 22c and a distal end part 22d provided on a distal end side of the insertion part 22a. The bendable part 22c performs a bending operation by operating an angle knob 22e of the operating part 22b. The distal end part 22d is directed in a desired direction by the bending operation of the bendable part 22c.

The distal end part 22d irradiates the observation target with illumination light generated from the light source device 24 and receives reflected light from the observation target to image the observation target. The operating part 22b is provided with an angle knob 22e and a still image acquisition instruction switch 22f. The still image acquisition instruction switch 22f is used to provide an instruction for acquisition of a still image of the observation target. The endoscope 22 may be a video flexible endoscope comprising an imaging sensor at the distal end part 22d, an endoscope provided with a zoom mechanism, an endoscope with an imaging sensor connected to a fiberscope with a small diameter at the insertion part 22a, or an endoscope with an external imaging sensor connected to a rigid endoscope.

As a route along which the endoscope is inserted into the subject, there is transnasal insertion in which the endoscope is inserted from a nose through a nasal cavity and a nasopharynx into an oropharynx, a hypopharynx, an esophagus, a stomach, and a duodenum, transoral insertion in which the endoscope is inserted from a mouth through an oral cavity into an oropharynx, a hypopharynx, an esophagus, a stomach, a duodenum, and a small intestine, or transanal insertion in which the endoscope is inserted from an anus into a rectum, a colon, and a small intestine. The image processing device 10 according to the present embodiment is particularly suitable in a case in which the endoscope 22 included in the endoscope system 21 is a flexible endoscope that is transnasally inserted into the subject. In the present specification, the subject refers to a subject into which the endoscope 22 is inserted. The observation target refers to a subject that is included in an angle of view of the endoscope 22 and is reflected in the medical image.

A still image acquisition instruction may be provided by using an operating device other than the still image acquisition instruction switch 22f. For example, a foot switch, a foot pedal, a touch panel, a gesture input device, a line-of-sight input device, or the like may be connected to the processor device 23 as the user interface 12, and the still image acquisition instruction may be provided in a case in which a specific operation is performed by the user on the user interface 12. In addition, in a case in which a specific voice input to the processor device 23 is recognized via a microphone as the user interface 12, the still image acquisition instruction may be provided.

The processor device 23 controls turning on and off the illumination light of the light source device 24, controls the operation with respect to the endoscope 22, controls the imaging by the endoscope 22, controls the image processing such as correction processing and enhancement processing on the endoscope image captured by the endoscope 22, and controls the transmission of the endoscope image to the image processing device 10.

The light source device 24 comprises a light source unit and a light source controller. The light source unit emits illumination light. The light source controller controls the operation of the light source unit. The light source unit is a light source such as a laser diode, a light emitting diode (LED), a xenon lamp, or a halogen lamp, and emits illumination light with which the observation target is illuminated. The light source unit may be built in the endoscope 22. The light source controller controls turning on and off and light emission amount of each light source constituting the light source unit.

An illumination optical system and an imaging optical system are provided at the distal end part 22d of the endoscope 22. The illumination light emitted by the light source unit is emitted from the distal end part 22d via an illumination lens of the illumination optical system. The imaging optical system includes an objective lens and an imaging sensor. Reflected light from the observation target irradiated with the illumination light is incident into the imaging sensor via the objective lens. As a result, an image of the observation target is formed on the imaging sensor, and an image signal is output from the imaging sensor. The imaging sensor may be provided in the distal end part 22d, may be connected to the endoscope 22 which is a fiberscope, or may be externally attached to the endoscope 22 which is a rigid endoscope.

The imaging sensor is a complementary metal oxide semiconductor (CMOS) sensor, a charge-coupled device (CCD) sensor, or the like. The processor device 23 generates a medical image based on the image signal output by the imaging sensor. The medical image generated by the processor device 23 is output to the image acquisition unit 30 of the image processing device 10. The medical image transmitted from the endoscope system 21 to the image processing device 10 is a series of time-sequentially continuous video images captured during endoscopy. The medical image may be transmitted from the endoscope system 21 to the database 11 for storage, and the medical image may be transmitted from the database 11 to the image processing device 10.

The database 11 is a storage, a file server, a cloud storage, or the like that stores a medical image such as an endoscope image. The database 11 may be a part of a system that directly or indirectly cooperates with the image processing device 10, such as hospital information systems (so-called HIS) or picture archiving and communication systems (PACS).

Hereinafter, a flow in which the image processing device 10 performs image processing on the medical image, outputs the observation support information, and notifies the user of the information via the user interface 12 will be described. The image acquisition unit 30 acquires the medical image by receiving the medical image from the endoscope system 21 which is the modality 20 or the database 11. The image acquisition unit 30 transmits the medical image to the observation target identification unit 40.

The observation target identification unit 40 inputs the medical image to an observation target identification algorithm provided in the observation target identification unit 40, and outputs observation target identification information. The observation target identification algorithm identifies an observation support target part, which is a part of the observation target included in the medical image, and outputs observation target identification information indicating the observation support target part. In addition, in a case of identifying that the medical image is out of an observation support target, the observation target identification unit 40 outputs the observation target identification information of "out of the observation support target". In the present specification, outputting various kinds of information includes generating various kinds of information.

The observation support target part is a part of an anatomical part of the body of the subject as the observation target. Specifically, the observation support target part is a nasal cavity, a nasopharynx, an oropharynx, a hypopharynx, a larynx, a trachea, a main bronchus, a lobar bronchus, a segmental bronchus, a subsegmental bronchus, an oral cavity, an esophagus, a stomach, a duodenum, a jejunum, a ileum, a cecum, an ascending colon, a transverse colon, a descending colon, a sigmoid colon, a rectum, or the like. The nasopharynx is also called an epipharynx. In a case in which the observation target identification algorithm identifies that the observation target included in the medical image is any of the observation support target parts, the observation target identification algorithm outputs the observation support target part as the observation target identification information.

In the present embodiment, it is preferable to output a nasal cavity, a nasopharynx, an oropharynx, a hypopharynx, a larynx, a trachea, or an esophagus as the observation support target part. In this case, in response to an input of a medical image in which a part other than these parts is the observation target, the observation target identification algorithm outputs "out of the observation support target". In this way, in a case in which the observation support target part is limited, the observation target identification algorithm outputs "nasopharynx" as the observation target identification information in a case in which it is identified that the observation target included in the medical image is the nasopharynx, and the observation target identification algorithm outputs "out of the observation support target" as the observation target identification information in a case in which it is identified that the observation target included in the medical image is the descending colon.

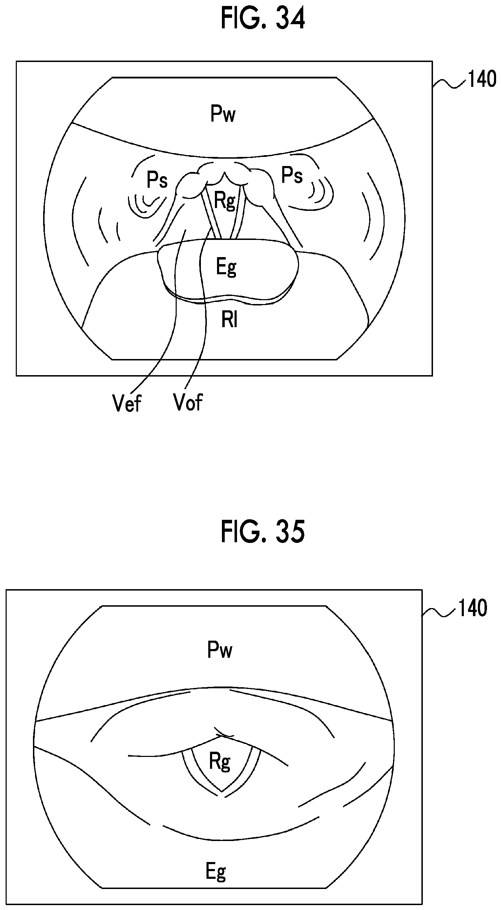

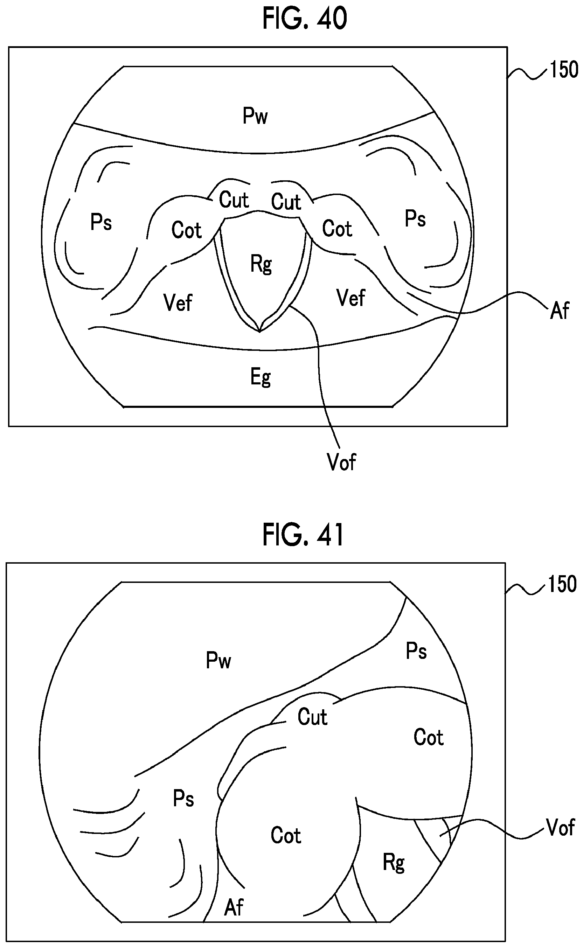

The larynx is included in a medical image in which the observation target is the oropharynx or the hypopharynx (see Figs. 34, 40, and the like described below). In a case in which the observation target identification algorithm identifies "larynx", an output of "oropharynx" or "hypopharynx" may be made. In addition, in a case in which the observation target identification algorithm identifies the trachea, the main bronchus, the lobar bronchus, the segmental bronchus, or the subsegmental bronchus, an output of "trachea" may be made. Further, as will be described below, the part output to the observation target identification unit 40 as the nasal cavity, nasopharynx, oropharynx, hypopharynx, larynx, trachea, or esophagus as the observation support target part may be narrowed to a more limited range of these parts.

In a case in which the observation target included in the medical image is identified as not the observation support target part, the observation target identification algorithm outputs that the medical image is out of the observation support target as the observation target identification information. The medical image output as being out of the observation support target by the observation target identification algorithm includes a medical image in which a foreign substance such as food or saliva is included in the observation target, a medical image in which the observation target is out of focus, a medical image in which the observation target is blurred, and a medical image in which the observation target includes halation, in addition to a medical image in which a part other than the observation support target part is an observation target. The observation target identification algorithm outputs "out of the observation support target" in response to such input of the medical image unsuitable for observation. The food as a foreign substance is residual food or swallowing food for examination. The saliva as a foreign substance is saliva accumulated in an anatomical part such as a pyriform sinus.

The observation target identification algorithm, and a position determination algorithm and an operation support algorithm described below may be machine learning or may be an algorithm that identifies a medical image by pattern matching. The machine learning includes decision trees, support vector machines, random forests, regression analysis, supervised learning, semi-unsupervised learning, unsupervised learning, reinforcement learning, deep learning, deep reinforcement learning, neural networks, convolutional neural networks, adversarial generative networks, and the like.

In the present embodiment, the image processing device 10 is suitable for a case in which the observation target identification algorithm as the machine learning, and the position determination algorithm and the operation support algorithm described below are convolutional neural networks. In addition, a part of the position determination algorithm and the operation support algorithm described below may be used as a convolutional neural network. In the present specification, in a case in which the observation target identification algorithm and the observation support algorithm described below are machine learning, the observation target identification algorithm or the observation support algorithm before learning is called a learning model, and the observation target identification algorithm or the observation support algorithm after learning is called a trained model.

Image data for training a learning model is called a learning image. The learning image is also called teacher data. The learning image includes a ground truth image, such as a ground truth image of the observation support target part, a ground truth image described below in which the observation target is an "appropriate" observation position, and a ground truth image in which a specific region included in the observation target is classified.

It is preferable that the observation target identification algorithm is a trained model that has been trained using a learning image including the medical image in which a nasal cavity, a nasopharynx, an oropharynx, a hypopharynx, a larynx, a trachea, or an esophagus is included in an observation target. For example, a convolutional neural network such as VGGNet, such as VGG16 or VGG19, or ResNet is suitable.

In a case in which the supervised learning or the semi-supervised learning is applied as the observation target identification algorithm, the ground truth image is a medical image showing the nasal cavity, nasopharynx, oropharynx, or hypopharynx that has been determined by the doctor as being suitable for observation. In addition, the ground truth image that is out of the observation support target includes an image captured outside the body of the subject, an image in which the doctor has determined that the observation target includes a foreign substance such as food or saliva, an image in which the doctor or an existing device that performs blur-shake determination has determined that the observation target includes blurriness or shake, an image in which a characteristic structure necessary for identifying the observation support target is not shown because it is captured too close to the observation target, an image in which the observation target is too dark to be distinguished, an image in which the observation target includes a large amount of halation, and the like. The halation is a region that is unsuitable for observation due to overexposure of the image due to strong light such as reflected light of illumination light from the observation target being incident into the imaging sensor. In addition, a medical image in which a determination is made by the doctor or an existing device that the subject is in the middle of performing a specific motion such as swallowing or coughing may be used as the ground truth image that is out of the observation support target. In addition, a medical image in which a part other than the nasal cavity, nasopharynx, oropharynx, hypopharynx, larynx, trachea, or esophagus is the observation target may be included in the ground truth image that is out of the observation support target.

The observation support algorithm selection unit 50 selects, from a plurality of observation support algorithms, one specific observation support algorithm based on the observation target identification information. The observation support algorithm selection unit 50 transmits specific observation support algorithm selection information, which is information indicating which specific observation support algorithm is selected, to the observation support information generation unit 60. The observation support algorithm is an algorithm in which the observation support information generation unit 60 outputs the observation support information, which will be described below, by inputting the medical image.

The observation support algorithm includes a nasal cavity observation support algorithm, a nasopharyngeal observation support algorithm, an oropharyngeal observation support algorithm, a hypopharyngeal observation support algorithm, an erroneous insertion observation support algorithm, and an observation support algorithm for out-of-observation-support-target. The observation support algorithm selection unit 50 selects one observation support algorithm among these observation support algorithms as the specific observation support algorithm.

Which observation support algorithm the observation support algorithm selection unit 50 selects as the specific observation support algorithm based on the observation target identification information is preferably set in advance. Specifically, the observation target identification information indicating the observation support target part or indicating that the medical image is out of the observation support target and the observation support algorithm are stored in association with each other, and the observation support algorithm selection unit 50 selects the specific observation support algorithm based on the association. For example, in a case in which the observation support target part as the observation target identification information is "nasal cavity", the observation support algorithm selection unit 50 selects the nasal cavity observation support algorithm as the specific observation support algorithm, and transmits information for providing an instruction for use of the nasal cavity observation support algorithm, to the observation support information generation unit 60, as the specific observation support algorithm selection information.

In addition, in a case in which the observation target identification information of "out of the observation support target" is output, the observation support algorithm selection unit 50 selects the observation support algorithm for out-of-observation-support-target as the specific observation support algorithm, and transmits information for providing an instruction for use of the observation support algorithm for out-of-observation-support-target, to the observation support information generation unit 60, as the specific observation support algorithm selection information.

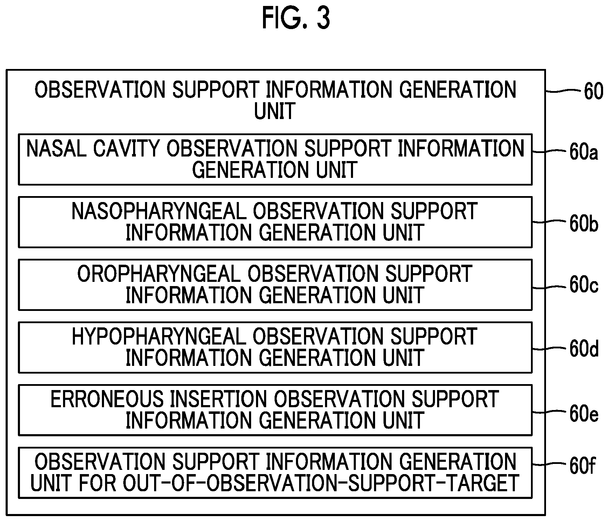

The observation support algorithm is included in the observation support information generation unit 60. As shown in Fig. 3, the observation support information generation unit 60 comprises a nasal cavity observation support information generation unit 60a, a nasopharyngeal observation support information generation unit 60b, an oropharyngeal observation support information generation unit 60c, a hypopharyngeal observation support information generation unit 60d, an erroneous insertion observation support information generation unit 60e, and an observation support information generation unit for out-of-observation-support-target 60f.

The nasal cavity observation support information generation unit 60a comprises a nasal cavity observation support algorithm. The nasopharyngeal observation support information generation unit 60b comprises a nasopharyngeal observation support algorithm. The oropharyngeal observation support information generation unit 60c comprises an oropharyngeal observation support algorithm. The hypopharyngeal observation support information generation unit 60d comprises a hypopharyngeal observation support algorithm. The erroneous insertion observation support information generation unit 60e comprises an erroneous insertion observation support algorithm. The observation support information generation unit for out-of-observation-support-target 60f comprises an observation support algorithm for out-of-observation-support-target.

In a case in which the observation support target part includes an alimentary canal, for example, an observation support information generation unit for a stomach may be provided in the observation support information generation unit 60, and a gastric observation support algorithm may be provided as the observation support algorithm. In this case, the observation support algorithm selection unit 50 can select the gastric observation support algorithm as the specific observation support algorithm.

The observation support information generation unit 60 inputs a medical image to the observation support algorithm selected as the specific observation support algorithm, and outputs the observation support information. The observation support information includes information for an operation instruction of the modality 20 to the operator who is the user, information indicating that the observation support has been stopped, information for an instruction to the subject, and the like.

Specifically, the observation support information includes endoscope position determination information indicating whether a position of the distal end part 22d of the endoscope 22 is appropriate or inappropriate, endoscope operation information indicating an operation method of the endoscope 22, subject position information for prompting change or confirmation of a position of the subject, observation support stop information indicating stop of the observation support, and the like. The observation support information includes insertion region information, insertion route information, nasopharyngeal position information, oropharyngeal region information, oropharyngeal region arithmetic information, hypopharyngeal region information, and hypopharyngeal region arithmetic information, which will be described below. The detailed contents of each observation support information will be described below. The observation support information generation unit 60 transmits the output observation support information to the notification controller 70.

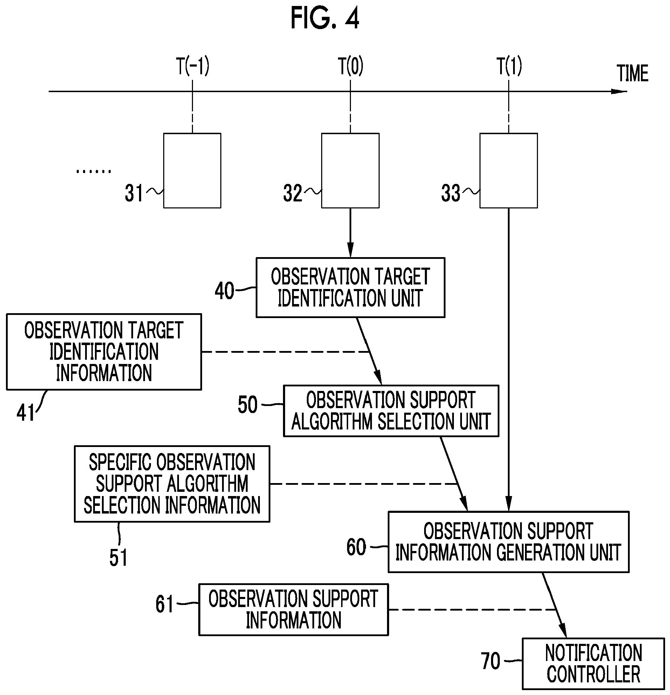

The same medical image as the medical image transmitted to the observation target identification unit 40 may be transmitted to the observation support information generation unit 60, and a medical image different from the medical image input to the observation target identification unit 40 may be transmitted to the observation support information generation unit 60. In a case in which a medical image different from the medical image transmitted to the observation target identification unit 40 is transmitted to the observation support information generation unit 60, the latest medical image is input to the observation support algorithm. This case will be described with reference to Fig. 4.

It is assumed that the image acquisition unit 30 acquires a medical image 31 at a time point T(-1), a medical image 32 at a time point T(0), and a medical image 33 at a time point T(1) in chronological order in a direction of an arrow indicating a time in Fig. 4. The medical image 31 is the latest medical image at the time point T(-1), the medical image 32 is the latest medical image at the time point T(0), and the medical image 33 is the latest medical image at the time point T(1).

At the time point T(0), the medical image 32 is transmitted to the observation target identification unit 40 and input to the observation target identification algorithm. Observation target identification information 41 output by the observation target identification algorithm is transmitted to the observation support algorithm selection unit 50, and the observation support algorithm selection unit 50 transmits specific observation support algorithm selection information 51 to the observation support information generation unit 60 based on the observation target identification information. It is assumed that the time has elapsed from the time point T(0) to the time point T(1) at this point in time and that the medical image 33 has been acquired. In this case, the observation support information generation unit 60 inputs the medical image 33, which is the latest medical image, to the specific observation support algorithm. Observation support information 61 output by the specific observation support algorithm is transmitted to the notification controller 70.

In this way, a notification of the observation support information can be provided in substantially real time by inputting the latest medical image to the observation support algorithm. In addition, the most recently acquired medical image (latest medical image) may be input to the position determination algorithm and the operation support algorithm, which will be described below. This is because, during endoscopy, medical images are continuously acquired in a very short period of time, so that the included observation target usually does not change significantly in the medical image determined by the position determination algorithm as being "appropriate" or "inappropriate" and the medical image as a target of the operation support, except in special cases such as a case in which the subject performs a specific motion such as swallowing or coughing.

The notification controller 70 performs a control of notifying of the observation support information. The observation support information is notified to the user via the user interface 12 as a guide image to be displayed on a display or a voice emitted from the speaker. In a case of displaying the guide image, the notification controller 70 generates the guide image using the observation support information, and performs a control of displaying the guide image on the display. In addition, the notification controller 70 generates voice instruction information using the observation support information, and performs a control of notifying of the observation support information as a voice from the speaker. In addition, as a method of the notification, the display of the guide image and the voice instruction information may be combined. The method of the notification is not limited to this.

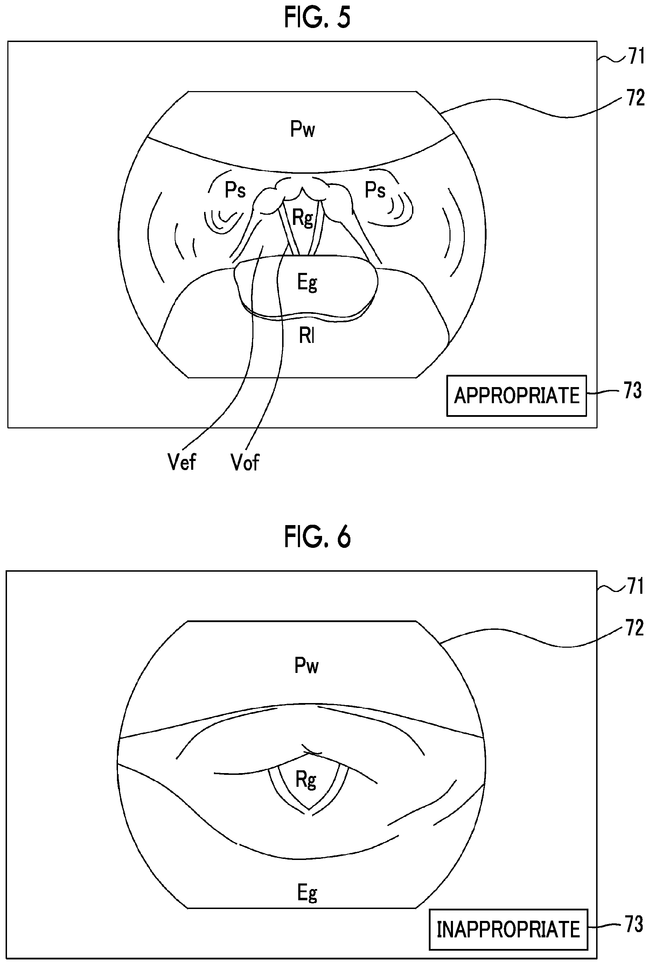

As a specific example, an example in which endoscope position determination information indicating that the position of the distal end part 22d of the endoscope 22 is appropriate or inappropriate is displayed on a guide image is described with reference to Fig. 5, Fig. 6, and Fig. 7.

In this example, it is assumed that the observation target identification algorithm outputs "oropharynx" as the observation support target part. In this case, the observation support algorithm selection unit 50 selects the oropharyngeal observation support algorithm as the specific observation support algorithm. The oropharyngeal observation support algorithm performs image processing on a medical image in which the observation target is an oropharynx (hereinafter, referred to as an "oropharyngeal image"), and outputs the endoscope position determination information indicating that the position of the distal end part 22d of the endoscope 22 is "appropriate" or "inappropriate", as the observation support information. In a case in which the oropharyngeal observation support algorithm outputs the endoscope position determination information of "appropriate", the notification controller 70 generates a guide image 71 shown in Fig. 5 and displays it on the display.

The notification controller 70 generates the guide image 71 by superimposing the observation support information on the medical image input to the observation support algorithm or the latest medical image, or by displaying these medical images and the observation support information in parallel. In the example of the guide image 71 shown in Fig. 5, a medical image 72 and an observation support information display field 73 are provided. In the example shown in Fig. 5, a message indicating the endoscope position determination information of "appropriate" is displayed in the observation support information display field 73.

The medical image 72 (oropharyngeal image) displayed in the guide image 71 illustrated in Fig. 5 includes an anatomical structure in the oropharynx, such as a tongue root Rl, an epiglottis Eg, a rima glottidis Rg, a vocal fold Vof, a pyriform sinus Ps, and a posterior pharyngeal wall Pw. Details about what kind of medical image is the oropharyngeal image output as "appropriate" will be described below.

On the other hand, in a case in which the oropharyngeal observation support algorithm outputs the endoscope position determination information of "inappropriate" indicating that the position of the distal end part 22d of the endoscope 22 is inappropriate, the notification controller 70 generates the guide image 71 shown in Fig. 6 and displays it on the display. The observation support information display field 73 is provided. In the example of the guide image 71 shown in Fig. 6, a message indicating the endoscope position determination information of "inappropriate" is displayed in the observation support information display field 73. The medical image 72 (oropharyngeal image) of the guide image 71 shown in Fig. 6 is an image captured too close to the larynx, and thus is determined as being "inappropriate".

In a case in which the oropharyngeal observation support algorithm outputs the endoscope position determination information of "appropriate" or "inappropriate", the notification controller 70 may perform a control of emitting a voice message such as "it is an appropriate position" or "it is an inappropriate position" or an alarm sound associated with each of "appropriate" and "inappropriate" from the speaker, as the voice instruction information. In addition, such a voice message may be emitted simultaneously with the guide image 71 shown in Fig. 6. Alternatively, a setting may be made in advance such that the voice message is emitted for both "appropriate" and "inappropriate" of the endoscope position determination information output by the oropharyngeal observation support algorithm.

Hereinafter, the notification of the observation support information in a case in which the observation target identification algorithm outputs the observation target identification information of "out of the observation support target" will be described. In this case, the observation support algorithm selection unit 50 selects the observation support algorithm for out-of-observation-support-target as the specific observation support algorithm. The observation support algorithm for out-of-observation-support-target performs image processing on a medical image identified as "out of the observation support target" (image that is out of the observation support target), and outputs the observation support stop information indicating that the observation support is stopped, as the observation support information.

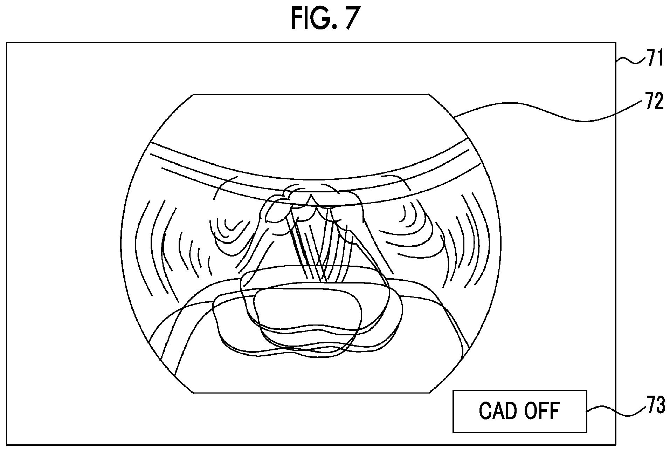

In this case, the notification controller 70 generates a guide image 71 shown in Fig. 7. In the example of the guide image 71 shown in Fig. 7, a message indicating the observation support stop information of "CAD OFF" indicating that the observation support (computer aided diagnosis/detection, CAD) is stopped is displayed in the observation support information display field 73. Since the guide image 71 illustrated in Fig. 7 is an image in which the medical image 72 is out of focus, the guide image 71 is a guide image generated by the notification controller 70 as a result of outputting "out of the observation support target" by the observation target identification algorithm, or outputting the observation support stop information by the observation support algorithm for out-of-observation-support-target.

With the above configuration, it is possible to output the observation support information according to a part of the observation target included in the medical image transmitted from the modality 20 or the database 11. In addition, it is possible to identify a medical image that is unsuitable for observation as being out of the observation support target, select a specific observation support algorithm according to a scene in which such a medical image is acquired, and then output the observation support information.

In the present embodiment, the observation target identification algorithm can identify a nasal cavity, a nasopharynx, an oropharynx, a hypopharynx, a larynx, a trachea, or an esophagus as the observation support target part. This configuration is particularly suitable for swallowing endoscopy in which it is required to perform an observation while holding the endoscope 22 at an appropriate position in a plurality of observation sites.

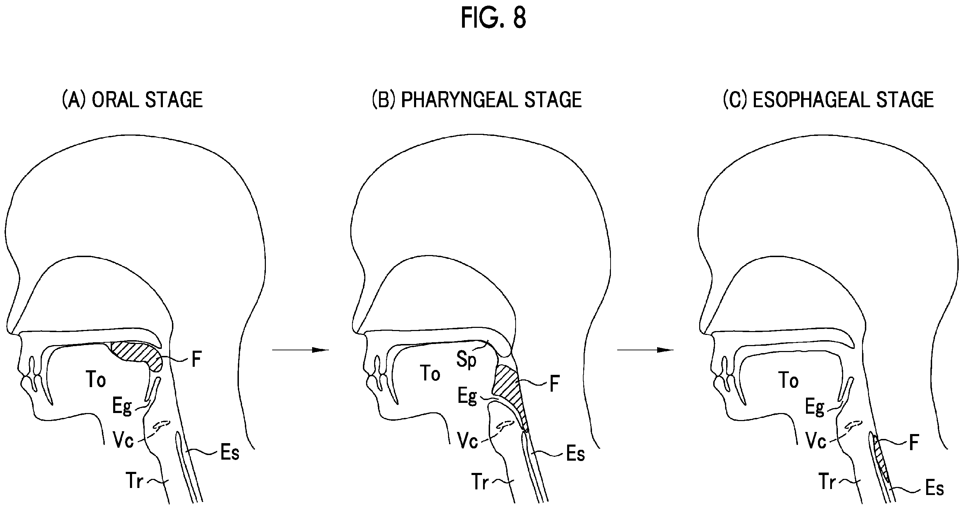

The swallowing endoscopy is one of methods used in an evaluation method (swallowing function evaluation examination) of dysphagia. The swallowing is a series of operations as shown in Fig. 8, in which food or drink is put into a mouth, chewed, swallowed, and sent to the esophagus. In the example of the normal swallowing movement shown in Fig. 8, a sequence transitions from an "oral stage" in which food F is transported from an oral cavity to a pharynx mainly by the movement of a tongue To, which is shown in (A) of Fig. 8, to a "pharyngeal stage" in which the food F is transported from the pharynx to an esophagus Es by the swallowing reflex, which is shown in (B) of Fig. 8, and then to an "esophageal stage" in which the food F is transported from the esophagus Es to the stomach by the peristaltic movement of the esophagus, which is shown in (C) of Fig. 8. In the case of swallowing, the food F is directed toward the esophagus Es and is not allowed to flow into a trachea Tr. Therefore, the epiglottis Eg, which plays a role of covering the trachea Tr, closes an entrance (glottis Vc) of the trachea Tr by the reflex movement. In addition, a soft palate Sp, which is a ceiling of the oral cavity, also moves backward to close a passage between the oral cavity and the nasal cavity, thereby preventing the food F from entering the nasal cavity.

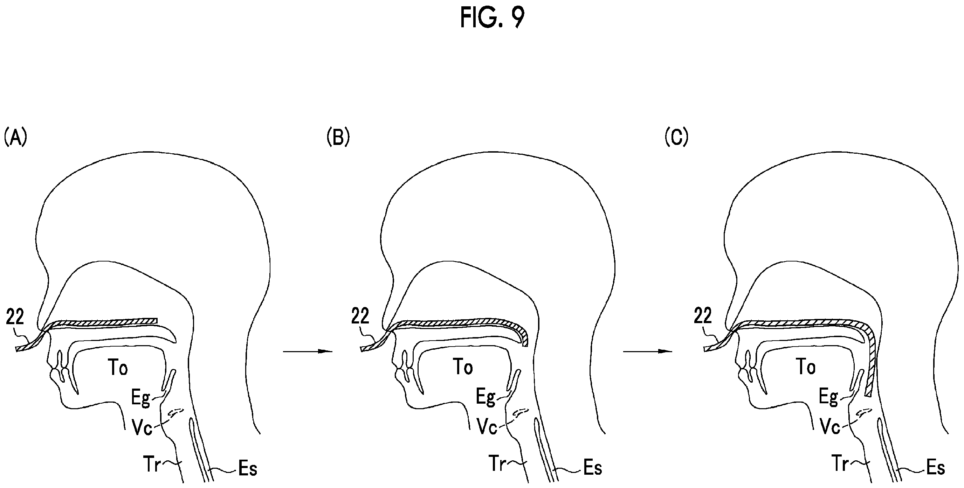

A condition in which food or drink cannot be swallowed properly due to aging or a disease of a nervous system is called dysphagia. In the swallowing endoscopy, it is recommended to observe a swallowing state from a plurality of observation positions in order to investigate the presence or absence of the dysphagia or the cause of the dysphagia. The plurality of observation positions are positions of three endoscopes 22 for observing the nasopharynx, oropharynx, and hypopharynx, as shown in (A) of Fig. 9, (B) of Fig. 9, and (C) of Fig. 9. In a case in which the endoscope 22 is transnasally inserted and the observation is performed in the order of the nasopharynx, oropharynx, and hypopharynx, the distal end part 22d is moved in the order of (A) of Fig. 9, (B) of Fig. 9, and (C) of Fig. 9. The observation position of the nasopharynx is as shown in (A) of Fig. 9. The observation position of the oropharynx is as shown in (B) of Fig. 9. The observation position of the hypopharynx is as shown in (C) of Fig. 9.

For appropriate diagnosis, it is necessary to dispose the distal end part 22d for each part involved in swallowing, as shown in (A) of Fig. 9, (B) of Fig. 9, and (C) of Fig. 9. Textbooks, guidelines, and the like indicate an appropriate disposition position different for each such part. However, an appropriate procedure for operating the endoscope 22 for disposing the distal end part 22d at that position depends on the experience of the doctor, and an appropriate operation method of the endoscope 22 also differs depending on the subject. Therefore, it is difficult for anyone other than a skilled person to dispose the endoscope 22 at an appropriate observation position and to cause the endoscope 22 to approach an appropriate observation position.

According to the present embodiment, it is possible to automatically provide, for each part, support for a determination as to whether or not the distal end part 22d is appropriately disposed at a position for observing the nasopharynx, oropharynx, or hypopharynx, and support for an operation of the endoscope 22 for moving the distal end part 22d to each appropriate observation position. Therefore, the observation support can be provided such that even the doctor who has little experience in the swallowing endoscopy can perform the observation according to the guideline.

As described above, in the swallowing endoscopy, a result of a determination as to whether or not the current position is an appropriate position, and the content of an operating guide of the endoscope 22 to the appropriate position differ depending on the part to be observed by the user, so that it is preferable that a different observation support algorithm is selected for each part as in the present embodiment. In a case in which one observation support algorithm is used in the swallowing endoscopy, it is assumed that even a medical image showing an appropriate position for observing the oropharynx is determined as being inappropriate because the glottis is too far away for observing the hypopharynx. Similarly, it is assumed that even a medical image showing an appropriate position for observing the hypopharynx is determined as being inappropriate because the glottis is too close for observing the oropharynx. In the present embodiment, since one specific observation support algorithm is selected from a plurality of observation support algorithms after identifying the observation target, it is possible to prevent inconsistency in the determination results in a case in which one observation support algorithm is used.

In addition, with a configuration in which the specific observation support algorithm is selected after identifying the observation target, it is possible to automatically switch between a guide in the direction in which the endoscope 22 advances toward a direction in which the glottis is observed in a near view (backward direction), and a guide in a direction in which the endoscope 22 is pulled forward toward a direction in which the glottis is observed in a distant view (forward direction), depending on whether the observation is intended for the oropharynx or the hypopharynx. According to the present embodiment, it is possible to perform the most appropriate observation support according to the observation purpose of the user.

Further, in addition to a period of time during which the distal end part 22d is finely adjusted to an appropriate position for observing the nasopharynx, oropharynx, or hypopharynx, the automatic switching to the most appropriate observation support algorithm can be performed during a period of time in which the distal end part 22d is in the nasal cavity until the endoscope 22 is moved from an anterior nostril to the nasopharynx.

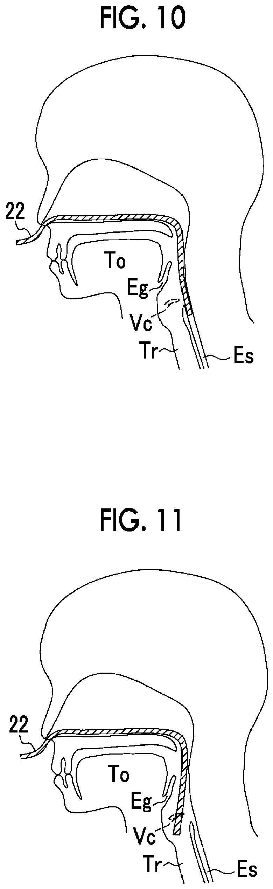

In addition, as shown in Figs. 10 and 11, in a case in which the endoscope 22 is erroneously inserted into the esophagus Es (Fig. 10) or the trachea Tr (Fig. 11), it is necessary to immediately pull the endoscope 22 forward and return it to the pharynx. Even in such a case, it is possible to automatically switch to the most appropriate observation support algorithm and to perform the observation support for pulling out the endoscope 22.

The observation target identification algorithm that identifies the observation support target part is preferably a trained model that has been trained using, as a ground truth image, a medical image showing the nasal cavity, nasopharynx, oropharynx, or hypopharynx that has been determined by the doctor as being suitable for observation. In addition, in a case of generating such a trained model, it is more preferable to use, as a learning image, a series of video images in which the observation target is switched to the nasal cavity, nasopharynx, oropharynx, and hypopharynx in the order in which the endoscope 22 is inserted transnasally. In addition, it is more preferable that the learning image includes a series of video images in which the observation target is switched in the order in which the endoscope 22 is removed, such as the hypopharynx, oropharynx, nasopharynx, and nasal cavity. Further, it is still more preferable that the learning image includes a series of video images in which the observation target is switched in the order in which the endoscope 22 is transorally inserted, such as the oral cavity, oropharynx, and hypopharynx. In this way, by generating a trained model using the learning image in which the order of the parts through which the endoscope 22 passes for the insertion or removal is time-sequentially weighted, it is possible to improve an accuracy of identification of the observation support target part of the observation target identification algorithm.

An example of the observation support information output by the observation support algorithm will be described below. The observation support information includes endoscope position determination information, endoscope operation information, subject position information, and observation support stop information.

The endoscope position determination information is information indicating that the position of the distal end part 22d of the endoscope 22 is "appropriate" or "inappropriate". The endoscope position determination information is information output by an oropharyngeal position determination algorithm and a hypopharyngeal position determination algorithm, which will be described below, among the observation support algorithms. A specific example of a notification of the endoscope position determination information in the oropharynx and the hypopharynx will be described below.

The endoscope operation information is information indicating an operation method of the endoscope. Specifically, it is information indicating a moving direction and/or a moving amount of the distal end part 22d of the endoscope 22. The moving direction of the distal end part 22d includes a right direction, a left direction, an upward direction, a downward direction, a backward direction, a forward direction, a right turn, or a left turn. The observation support algorithm that outputs the endoscope operation information is a nasal cavity operation support algorithm, a nasopharyngeal operation support algorithm, an oropharyngeal operation support algorithm, a hypopharyngeal operation support algorithm, and an erroneous insertion operation support algorithm, which will be described below. A specific example of a notification of the endoscope operation information in the nasal cavity, nasopharynx, oropharynx, hypopharynx, esophagus, and trachea will be described below.

The subject position information is information for prompting the user to change or confirm the position of the subject, which is recommended for obtaining a medical image suitable for observation. Specifically, it is information for providing an instruction to change a position or posture of the subject, such as an angle of bending (forward bending), extension (backward bending) or rotation of the head and neck, an angle of a jaw with respect to a trunk, and an orientation of the trunk, or for prompting confirmation of the position or posture. The observation support algorithm that outputs the subject position information is an oropharyngeal operation support algorithm and a hypopharyngeal operation support algorithm, which will be described below. In the endoscopy, the operation of the endoscope 22 by the operator may not be enough to hold the distal end part 22d at an appropriate observation position. Therefore, by notifying of the subject position information, it is possible to support the doctor to make the posture of the subject appropriate or to prompt the subject himself/herself to make the posture appropriate and to move the distal end part 22d to an appropriate observation position.

The observation support stop information is information indicating that the observation support is stopped. The observation support algorithm that outputs the observation support stop information is an operation support algorithm for out-of-observation-support-target, which will be described below. As the notification of the observation support stop information, a message indicating the observation support stop information such as "CAD OFF" is displayed in the guide image (see Fig. 7). In addition, the notification is performed by stopping the notification of the observation support information such as the endoscope position determination information, the endoscope operation information, and the subject position information.

Hereinafter, for a case in which the observation support target part identified from the medical image is the nasal cavity, nasopharynx, oropharynx, hypopharynx, esophagus, or trachea, and a case in which the medical image is identified as being out of the observation support target, the used observation support algorithm, the observation support information output by the observation support algorithm, and the method of notifying of the observation support information are respectively described with specific examples.

First, the observation support algorithm will be described. The observation support algorithm includes the position determination algorithm and the operation support algorithm, and an algorithm to be used is switched by the specific observation support algorithm selected according to the observation target identification information. Specifically, in a case in which the observation target identification information is output as "nasal cavity", "nasopharynx", "esophagus", "trachea", or "out of the observation support target", the operation support algorithm is used as the observation support algorithm. On the other hand, in a case in which the observation target identification information is output as "oropharynx" or "hypopharynx", the position determination algorithm and the operation support algorithm are used as the observation support algorithm. Hereinafter, each part will be specifically described.

In a case in which the observation support target part is the nasal cavity, the nasal cavity observation support algorithm provided in the nasal cavity observation support information generation unit 60a is selected as the specific observation support algorithm. The nasal cavity observation support algorithm is the operation support algorithm. The operation support algorithm used in a case in which the observation support target part is the nasal cavity is called a nasal cavity operation support algorithm.

In a case in which the observation support target part is the nasopharynx, the nasopharyngeal observation support algorithm provided in the nasopharyngeal observation support information generation unit 60b is selected as the specific observation support algorithm. The nasopharyngeal observation support algorithm is the operation support algorithm. The operation support algorithm used in a case in which the observation support target part is the nasopharynx is called a nasopharyngeal operation support algorithm.

In a case in which the observation support target part is the oropharynx, the oropharyngeal observation support algorithm provided in the oropharyngeal observation support information generation unit 60c is selected as the specific observation support algorithm. The oropharyngeal observation support algorithm is configured of a combination of the position determination algorithm and the operation support algorithm. The position determination algorithm used in a case in which the observation support target part is the oropharynx is called an oropharyngeal position determination algorithm. The operation support algorithm used in a case in which the observation support target part is the oropharynx is called an oropharyngeal operation support algorithm.

In a case in which the observation support target part is the hypopharynx, the hypopharyngeal observation support algorithm provided in the hypopharyngeal observation support information generation unit 60d is selected as the specific observation support algorithm. The hypopharyngeal observation support algorithm is configured of a combination of the position determination algorithm and the operation support algorithm. The position determination algorithm used in a case in which the observation support target part is the hypopharynx is called a hypopharyngeal position determination algorithm. The operation support algorithm used in a case in which the observation support target part is the hypopharynx is called a hypopharyngeal operation support algorithm.

In a case in which the observation support target part is the esophagus or trachea, the erroneous insertion observation support algorithm provided in the erroneous insertion observation support information generation unit 60e is selected as the specific observation support algorithm. The erroneous insertion observation support algorithm is the operation support algorithm. The operation support algorithm used in a case in which the observation support target part is the esophagus or trachea is called an erroneous insertion operation support algorithm.

In a case in which the medical image is identified as being out of the observation support target, the observation support algorithm for out-of-observation-support-target provided in the observation support information generation unit for out-of-observation-support-target 60f is selected as the specific observation support algorithm. The observation support algorithm for out-of-observation-support-target is the operation support algorithm. The operation support algorithm used in a case in which the medical image is identified as being out of the observation support target is called an operation support algorithm for out-of-observation-support-target.

Hereinafter, observation support information output by the nasal cavity operation support algorithm and a notification of the observation support information will be described. The nasal cavity operation support algorithm is an operation support algorithm for performing the observation support by indicating an appropriate insertion route of the endoscope 22 from the anterior nostril to the nasopharynx.

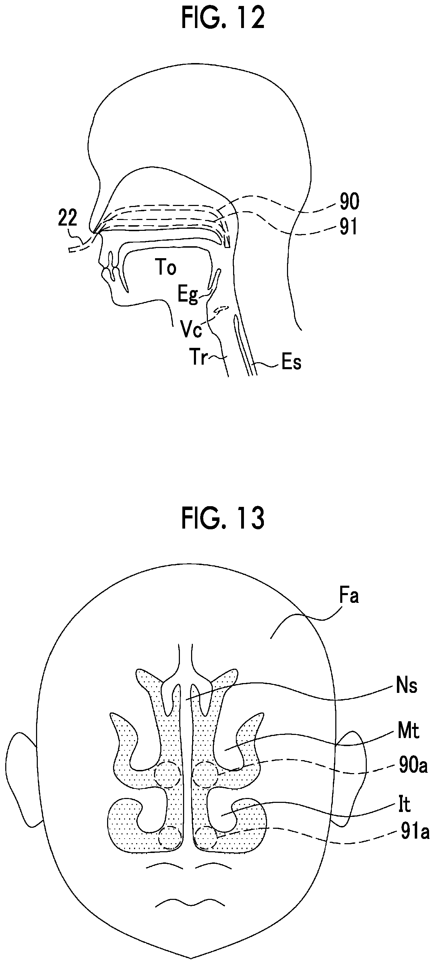

There are two transnasal insertion routes for the endoscope 22, one of which is an upper route 90 from the anterior nostril to a posterior nostril through a region of a common nasal meatus that is surrounded by a middle turbinate Mt, a nasal septum Ns, and an inferior turbinate It, as shown in Fig. 12. The other is a lower route 91 from the anterior nostril to the posterior nostril through a region of the common nasal meatus that is surrounded by the inferior turbinate It, the nasal septum Ns, and a nasal floor Nf, as shown in Fig. 12.

A region where the endoscope 22 is inserted in the upper route in a case in which a face Fa of a human is viewed from the front is a region 90a surrounded by the middle turbinate Mt, the nasal septum Ns, and the inferior turbinate It as shown in Fig. 13. A region where the endoscope 22 is inserted in the lower route is a region 91a surrounded by the inferior turbinate It, the nasal septum Ns, and the nasal floor Nf as shown in Fig. 13. In Fig. 13, a region as a "nasal cavity" through which the endoscope 22 may pass is shown by a dot pattern. The nasal cavity includes the common nasal meatus, and a superior nasal meatus, a middle nasal meatus, and an inferior nasal meatus which are continuous with the common nasal meatus and unsuitable for insertion of the endoscope 22.

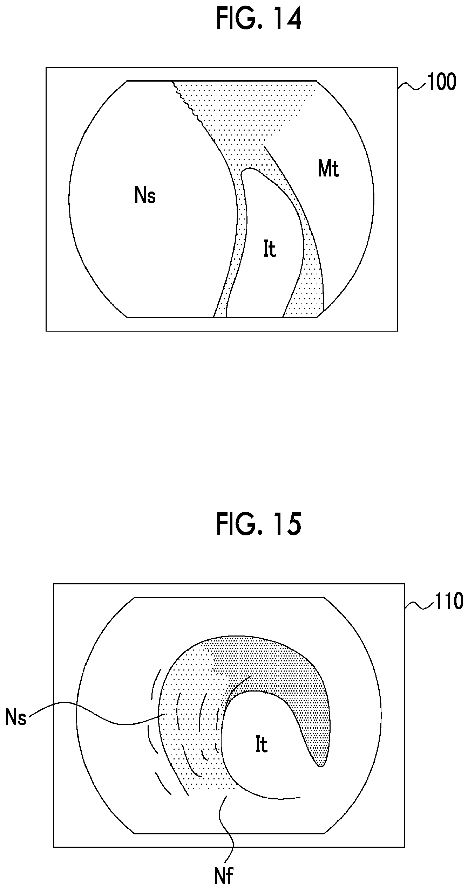

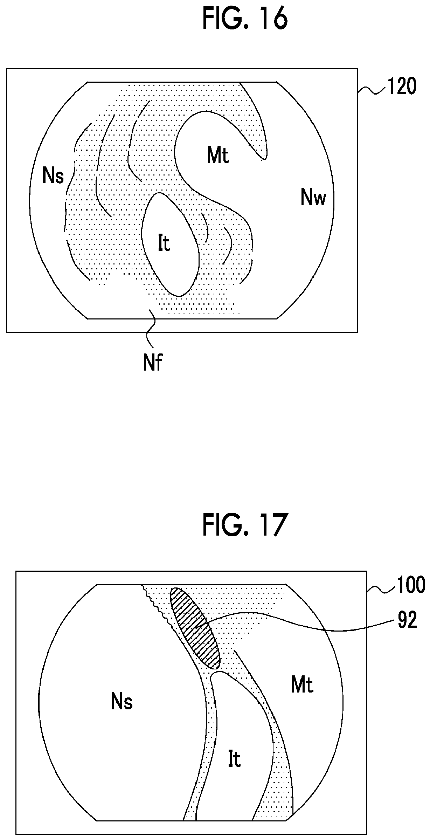

Fig. 14 shows an example of a medical image (hereinafter, referred to as an upper route image 100) captured while passing through the upper route. In addition, Fig. 15 shows an example of a medical image (hereinafter, referred to as a lower route image 110) captured while passing through the lower route. Further, Fig. 16 shows an example of a medical image (hereinafter, referred to as an anterior nasal image 120) in which both an upper route insertion region and a lower route insertion region, which will be described below, are included in the observation target. The anterior nasal image is a medical image captured at a position close to the anterior nostril in the region of the nasal cavity.

Fig. 14, Fig. 15, and Fig. 16 are examples of medical images in which the left nasal cavity of the subject is captured, in which the upper side of the paper shows the head side of the subject, the lower side of the paper shows the tail side of the subject, the right side of the paper shows the left side of the subject, and the left side of the paper shows the right side of the subject. The observation target identification algorithm as a trained model is trained using the upper route image 100 as shown in Fig. 14, the lower route image 110 as shown in Fig. 15, and the anterior nasal image 120 as shown in Fig. 16 as a learning image of "nasal cavity". In addition, the observation target identification algorithm as a trained model is trained using a medical image (upper route image and/or lower route image) captured while passing the upper route and the lower route for each of the right nasal cavity and the left nasal cavity.

Hereinafter, a case in which the upper route image is input to the nasal cavity operation support algorithm will be described. In a case in which the upper route image as shown in Fig. 14 is input, the nasal cavity operation support algorithm first outputs, as insertion region information, an upper route insertion region 92, which is a region surrounded by the middle turbinate Mt, the nasal septum Ns, and the inferior turbinate It and suitable for insertion of the endoscope 22, as shown in Fig. 17.

It is preferable that the nasal cavity operation support algorithm that outputs the upper route insertion region is a trained model that performs segmentation on the medical image and outputs the upper route insertion region. It is preferable that the trained model for outputting the upper route insertion region is generated by performing learning using a learning image including a ground truth image in which the upper route insertion region is classified in advance by the doctor. As a learning model applied to the generation of such a trained model, it is preferable to apply a pyramid scene parsing network (PSPnet), and a learning model suitable for other segmentation, such as spatial pyramid pooling network (SPPnet) may be used. The nasal cavity operation support algorithm may be a trained model generated by training a learning model to which unsupervised learning is applied.

Next, the nasal cavity operation support algorithm outputs the insertion route information using the insertion region information indicating the upper route insertion region. The insertion route information is information used for smoothly inserting the insertion part 22a of the endoscope 22 into the body. Specifically, the insertion route information is coordinate information of a center position of the upper route insertion region in a case in which the upper route image is input to the nasal cavity operation support algorithm. In addition, the insertion route information may be the area or width of the upper route insertion region.

The coordinate of the center position of the upper route insertion region is the center of gravity or the geometric center of the upper route insertion region. The geometric center of the upper route insertion region is obtained, for example, by a general method of calculating the geometric center of a polygon by taking coordinates of any number of points from pixels constituting a peripheral edge of the upper route insertion region. For example, in a case in which the number of pixels in the upper route insertion region is k, and the coordinates of an i-th pixel are (x_i,y_i) with any point in the upper route insertion region as an origin, a value obtained by dividing the sum of each coordinate by the number of pixels k is defined as the geometric center. That is, it can be calculated as (x-coordinate of geometric center of upper route insertion region) = Σ(x_i)/k and (y-coordinate of geometric center of upper route insertion region) = Σ(y_i)/k. A method of obtaining the coordinate information of the center position of the upper route insertion region is not limited to this. For example, a process of surrounding the upper route insertion region with a rectangle may be performed, and the coordinates of the center position of the rectangle may be used. The coordinates of any two points may be taken from the pixels constituting the upper route insertion region, and a center point of the longest distance between the two points may be used as the coordinate of the center position.

The area of the upper route insertion region is calculated by a method of calculating the area of the region classified as the upper route insertion region by segmentation. The width of the upper route insertion region refers to the longest distance between the coordinate of any two points taken from the pixels constituting the upper route insertion region.

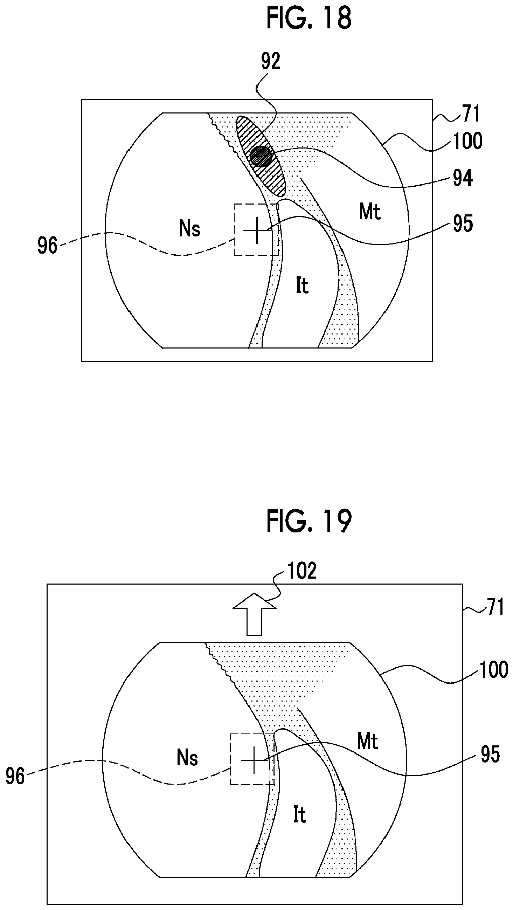

Hereinafter, an example of notifying of the observation support information will be described. First, an example will be described in which the insertion route information is used as the coordinates of the center position of the upper route insertion region. The nasal cavity operation support algorithm outputs the upper route insertion region 92 as shown in Fig. 17, and then outputs the coordinates of the center position of the upper route insertion region 92 as the insertion route information and transmits the output to the notification controller 70. The notification controller 70 generates the guide image 71 as shown in Fig. 18 and performs a control of displaying the guide image 71 on the display.

In the example of the guide image 71 shown in Fig. 18, an insertion route guide mark 94 indicating the coordinates of the center position of the upper route insertion region 92, an image center guide mark 95 (cross mark) indicating the center position of the upper route image 100, and a guide frame 96 that makes the image center guide mark 95 easily visible are displayed. In this case, the user performs the operation of the endoscope 22 such that a position of the insertion route guide mark 94 is aligned with the image center guide mark 95, whereby the distal end part 22d can be set to an ideal insertion position.

The ideal insertion position in the upper route is a position where the center position of the upper route insertion region is close to the center position of the medical image and the width of the upper route insertion region extends in the vertical direction of the medical image. Specifically, in a medical image observed in real time, the ideal insertion position is a position of the distal end part 22d where the middle turbinate Mt and the nasal septum Ns are on the left and right sides of the medical image and the inferior turbinate It is on the lower side of the medical image.

In a case in which the distal end part 22d of the endoscope 22 is disposed at an ideal insertion position where the insertion route guide mark 94 and the image center guide mark 95 overlap, the user can smoothly insert the distal end part 22d by causing the distal end part 22d to advance toward the back side of the nasal cavity (pushing the distal end part 22d). Therefore, by displaying the guide image 71 in which the insertion route guide mark 94 and the image center guide mark 95 are displayed as shown in Fig. 18, it is possible to perform the observation support for the user to smoothly insert the distal end part 22d. As a result, the pain of the subject due to the insertion of the endoscope 22 can be reduced.

Although the upper route insertion region 92 is depicted in Fig. 18, the upper route insertion region 92 may or may not be displayed on the guide image 71. The image center guide mark 95 is displayed at a fixed position of the guide image 71. In this case, the notification controller 70 notifies of the insertion route information transmitted from the nasal cavity operation support algorithm as the observation support information. In addition to the upper route insertion region, the guide image 71 may display a lower route insertion region, a glottis region, an epiglottis region, and a vocal fold region, which will be described below, as the insertion region information. In addition, the display or non-display of the insertion region information in the guide image 71 may be switched between display and non-display by operating a notification display switching button, which will be described below, or the operating part 22b.

The nasal cavity operation support algorithm may generate the endoscope operation information by using the insertion route information, which is the coordinates of the center position of the upper route insertion region, and notify of the endoscope operation information as the observation support information. In this case, the nasal cavity operation support algorithm outputs a vector indicating the moving direction and the moving amount for moving the distal end part 22d, as the endoscope operation information. The endoscope operation information as a vector is a direction and a distance from the coordinates of the center position of the upper route insertion region 92 to the coordinates of the center position of the upper route image 100. The calculation of the vector is performed by calculating a difference between information on the coordinates of the center position of the upper route insertion region 92 and information on the coordinates of the center position of the upper route image 100. The endoscope operation information output by the nasal cavity operation support algorithm is transmitted to the notification controller 70.

In this case, the notification controller 70 generates the guide image 71 as shown in Fig. 19 and performs a control of displaying the guide image 71 on the display. In the example of the guide image 71 shown in Fig. 19, the image center guide mark 95, the guide frame 96, and a guide arrow 102 are displayed. The guide arrow 102 is an arrow-shaped display indicating the moving direction and the moving amount for moving the distal end part 22d from the image center guide mark 95 (coordinates of the center position of the upper route insertion region) to the image center guide mark 95 (coordinates of the center position of the upper route image), which are generated based on the endoscope operation information. The guide arrow 102 may be displayed larger or smaller than the guide image 71 shown in Fig. 19 to indicate that the moving amount of the distal end part 22d is large or small. For example, the magnitude of the moving amount can be indicated by a length of the guide arrow 102. By displaying the guide image 71 as shown in Fig. 19, it is possible to indicate, to the user, the moving direction and the moving amount of the distal end part 22d by the guide arrow.

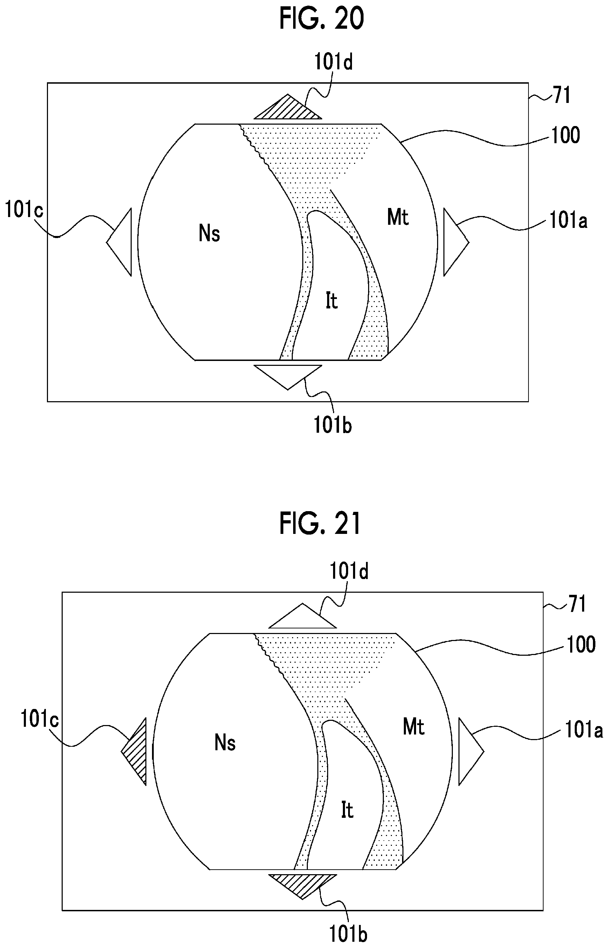

Another example of notifying of the endoscope operation information output by the nasal cavity operation support algorithm will be described. For example, in a case in which the upper route image as shown in Fig. 14 is input to the nasal cavity operation support algorithm, the notification controller 70 generates the guide image 71 as shown in Fig. 20. In the example of the guide image 71 shown in Fig. 20, guide direction display icons 101a, 101b, 101c, and 101d are displayed around the upper route image 100. In the guide image 71, the moving direction of the distal end part 22d is shown, for example, by making display modes of respective guide direction display icons different according to an ideal insertion route. Specifically, in a case in which the moving direction of the distal end part 22d is output as "upward direction" as the endoscope operation information, the guide direction display icons 101a, 101b, 101c, and 101d are displayed by making a color of the guide direction display icon 101d different from colors of the guide direction display icons 101a, 101b, and 101c, as shown in the example shown in Fig. 20. In the example shown in Fig. 20, the color of the guide direction display icon is different depending on the presence or absence of a diagonal line, and the endoscope 22 is operated in the upward direction.

In addition, in a case in which the moving direction of the distal end part 22d is set to "lower left direction" as the endoscope operation information, the guide direction display icons 101a, 101b, 101c, and 101d are displayed by making colors of the guide direction display icon 101c in the left direction and the guide direction display icon 101b in the downward direction different from colors of the guide direction display icon 101a in the right direction and the guide direction display icon 101d in the upward direction, as in the example of the guide image 71 shown in Fig. 21. The display mode of the guide direction display icon is not limited to this.

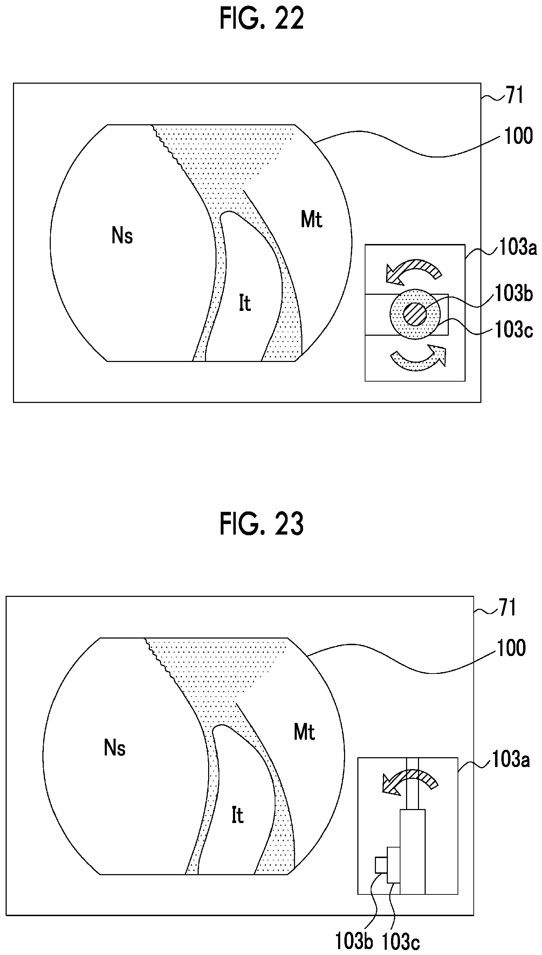

In addition, as the endoscope operation information, an endoscope operation support diagram showing an operation of the angle knob 22e for bending and moving the distal end part 22d may be displayed on the guide image 71. In the guide image 71 shown in Fig. 22, an endoscope operation support diagram 103a showing an operation of the angle knob 22e for causing the distal end part 22d to advance in "lower left direction" as the moving direction is displayed. The endoscope operation support diagram 103a shown in Fig. 22 shows an example of an operation instruction to rotate an outer lever 103b for bending the bendable part 22c in the left-right direction, and an inner lever 103c for bending the bendable part 22c in the up-down direction counterclockwise.

In the guide image 71 shown in Fig. 23, the endoscope operation support diagram 103a showing an operation of the angle knob 22e for "left turning" the distal end part 22d to as the moving direction is displayed. The endoscope operation support diagram 103a in the guide image 71 shown in Fig. 23 is a diagram drawn such that the angle knob 22e is disposed on the left side as viewed from the operator. The guide direction display icon, the arrow display, the guide arrow, and the endoscope operation support diagram may be combined and displayed on the guide image. With the above configuration, it is possible to automatically notify the user of the ideal insertion route of the endoscope in the nasal cavity. The moving direction and the moving amount of the endoscope 22 may be notified by a voice.

Hereinafter, an example of a notification of the observation support information in a case in which the insertion route information is the area or width of the upper route insertion region will be described. In this case, a display mode of the upper route insertion region may be changed according to the insertion route information output by the nasal cavity operation support algorithm. For example, for the area of the upper route insertion region, a first threshold value for displaying an insertion region and a second threshold value for displaying an insertion region smaller than the first threshold value for displaying an insertion region are provided. As a display mode of the upper route insertion region of the guide image 71 (for example, a display mode of the upper route insertion region 92 in Fig. 18), the upper route insertion region is displayed in blue in a case in which the area of the upper route insertion region is larger than the first threshold value for displaying an insertion region, the upper route insertion region is displayed in yellow in a case in which the upper route insertion region is equal to or less than the first threshold value for displaying an insertion region and larger than the second threshold value for displaying an insertion region, and the upper route insertion region is displayed in red in a case in which the upper route insertion region is equal to or less than the second threshold value for displaying an insertion region. That is, the display is switched to blue, yellow, and red in descending order of the area of the upper route insertion region. The first threshold value for displaying an insertion region and the second threshold value for displaying an insertion region can be set to optional values. In addition, the display mode of the upper route insertion region is not limited to this. Further, a first threshold value for displaying an insertion region and a second threshold value for displaying an insertion region may be set with respect to the width of the upper route insertion region. In this case, the notification controller 70 notifies of the insertion route information transmitted from the nasal cavity operation support algorithm as the observation support information.

The upper route image acquired during the insertion of the endoscope 22 may include an image in which the nasal cavity operation support algorithm cannot output the upper route insertion region. For example, the upper route insertion region may not be reflected in the upper route image acquired in real time because the insertion position is largely deviated from the ideal insertion position, or the upper route insertion region may be too small even though the upper route insertion region is reflected. The nasal cavity operation support algorithm may be used as a trained model by using a learning image in which such an upper route image in which the upper route insertion region is not reflected or an upper route image in which the upper route insertion region is too small is associated with the moving direction or the moving amount (that is, the endoscope operation information) of the distal end part 22d to the ideal insertion position. In this case, the nasal cavity operation support algorithm to which the upper route image in which the upper route insertion region is not reflected or the upper route image in which the upper route insertion region is too small is input outputs the endoscope operation information.

The nasal cavity operation support algorithm that outputs the upper route insertion region may be used as a first nasal cavity operation support algorithm, and the nasal cavity operation support algorithm that outputs the endoscope operation information in response to an input of the upper route image in which the upper route insertion region is not reflected or the upper route image in which the upper route insertion region is too small may be used as a second nasal cavity operation support algorithm. The latest medical image may be input to the first nasal cavity operation support algorithm and/or the second nasal cavity operation support algorithm.

In addition, in the learning of the nasal cavity operation support algorithm as the learning model, it is preferable to use a learning image in which the orientation (up, down, left, or right direction) of the upper route image input to the nasal cavity operation support algorithm is distinguished in advance. In addition, in the learning of the nasal cavity operation support algorithm, it is preferable that the parameters are updated in consideration of the orientation. In addition, in a case of associating the learning image with the ideal insertion position, it is preferable that the association is performed in consideration of the orientation. The same applies to the lower route image and the anterior nasal image described below.

With the above configuration, it is possible to prompt the operator who is inexperienced in the transnasal insertion of the endoscope to confirm that a route along which the endoscope can be easily inserted can be selected. In addition, there are individual differences in the upper route insertion region depending on an internal structure of the nasal cavity of the subject. With the above configuration, it is possible to prompt the operator to pay attention to the insertion of the endoscope and to prompt the operator to select the insertion route that reduces the pain of the subject because of the insertion of the endoscope, in a case in which the subject has a small upper route insertion region.

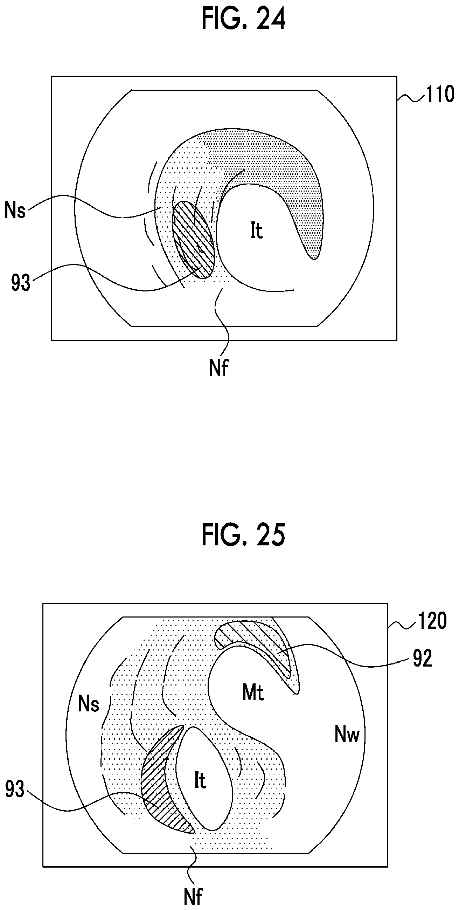

Hereinafter, a case in which the lower route image is input to the nasal cavity operation support algorithm will be described. In a case in which the lower route image as shown in Fig. 15 is input, the nasal cavity operation support algorithm first outputs, as insertion region information, a lower route insertion region 93, which is a region surrounded by the inferior turbinate It, the nasal septum Ns, and the nasal floor Nf and suitable for insertion of the endoscope 22, as shown in Fig. 24. As with the upper route insertion region (see Fig. 17), the lower route insertion region 93 is a region classified by the nasal cavity operation support algorithm as a trained model for performing segmentation.

Next, the nasal cavity operation support algorithm outputs the insertion route information using the insertion region information indicating the lower route insertion region. The insertion route information is coordinate information of a center position of the lower route insertion region in a case in which the lower route image is input to the nasal cavity operation support algorithm. In addition, the insertion route information may be the area or width of the lower route insertion region. The coordinate of the center position of the lower route insertion region is the center of gravity or the geometric center of the lower route insertion region. A calculation method of the center position of the lower route insertion region is the same as the calculation method of the center position of the upper route insertion region, and thus the description thereof will be omitted. In addition, a calculation method of the area or width of the lower route insertion region is also the same as the calculation method of the area or width of the upper route insertion region, and thus the description thereof will be omitted.

Since a method of notifying of the observation support information in a case in which the lower route image is input to the nasal cavity operation support algorithm is the same as in a case in which the upper route image is input to the nasal cavity operation support algorithm, detailed examples will be omitted. Briefly, in a case in which the insertion route information is the coordinates of the center position of the lower route insertion region, a control of displaying the guide image 71 in which the insertion route guide mark 94 and the image center guide mark 95 are displayed, as shown in Fig. 18, is performed using the insertion route information as the observation support information.

The ideal insertion position in the lower route is a position where the center position of the lower route insertion region is close to the center position of the medical image and the width of the lower route insertion region extends in the vertical direction of the medical image. Specifically, in a medical image observed in real time, the ideal insertion position is a position of the distal end part 22d where the inferior turbinate It and the nasal septum Ns are on the left and right sides of the medical image and the nasal floor Nf is on the lower side of the medical image.

In addition, in a case in which the insertion route information is the coordinates of the center position of the lower route insertion region, the nasal cavity operation support algorithm may output the endoscope operation information by using the insertion route information. The endoscope operation information is notified as the guide image 71 in which the image center guide mark 95 and the guide arrow 102 are displayed as shown in Fig. 19, the guide image 71 in which the guide direction display icon is displayed as shown in Fig. 20, or the guide image 71 in which the endoscope operation support diagram is displayed as shown in Figs. 21 and 22. The method of outputting the endoscope operation information is the same as in a case in which the upper route image is input to the nasal cavity operation support algorithm.

The method of outputting and notifying of the observation support information in a case in which the insertion route information is the area or width of the lower route insertion region or in a case in which the lower route image in which the lower route insertion region is not reflected or the lower route image in which the lower route insertion region is too small is input to the nasal cavity operation support algorithm is also the same as in a case in which the upper route image is input to the nasal cavity operation support algorithm.

Hereinafter, a case in which the anterior nasal image is input to the nasal cavity operation support algorithm will be described. In a case in which the anterior nasal image 120 as shown in Fig. 16 is input, the nasal cavity operation support algorithm first outputs, as the insertion region information, the upper route insertion region 92 surrounded by the middle turbinate Mt, the nasal septum Ns, and the inferior turbinate It and the lower route insertion region 93 surrounded by the inferior turbinate It, the nasal septum Ns, and the nasal floor Nf, which are suitable for insertion of the endoscope 22, as shown in Fig. 25. In the example of the anterior nasal image 120 shown in Fig. 25, a region (see Fig. 13) connected to the common nasal meatus on a side opposite to a nasal side wall Nw (that is, a side of the nasal septum Ns) is detected as the upper route insertion region 92. That is, the nasal cavity operation support algorithm outputs a region suitable for insertion of the endoscope 22 for both the upper route and the lower route, in response to an input of the anterior nasal image 120. In this case, the upper route insertion region 92 and the lower route insertion region 93 are each classified into different classes as a result of segmentation.

Next, the nasal cavity operation support algorithm outputs the insertion route information for each of the upper route insertion region and the lower route insertion region by using the classified insertion region information (upper route insertion region and lower route insertion region) in the input anterior nasal image. A method of outputting the insertion route information is the same as in a case of using the upper route image 100 or the lower route image 110, and thus the description thereof will be omitted.

Next, the nasal cavity operation support algorithm outputs the insertion route information for each of the upper route insertion region and the lower route insertion region. Further, the endoscope operation information is output using the insertion route information. A method of outputting the insertion route information and the endoscope operation information and a preferred embodiment thereof are the same as the method of outputting the insertion route information and the endoscope operation information using the upper route image or the lower route image, and thus the description thereof will be omitted.

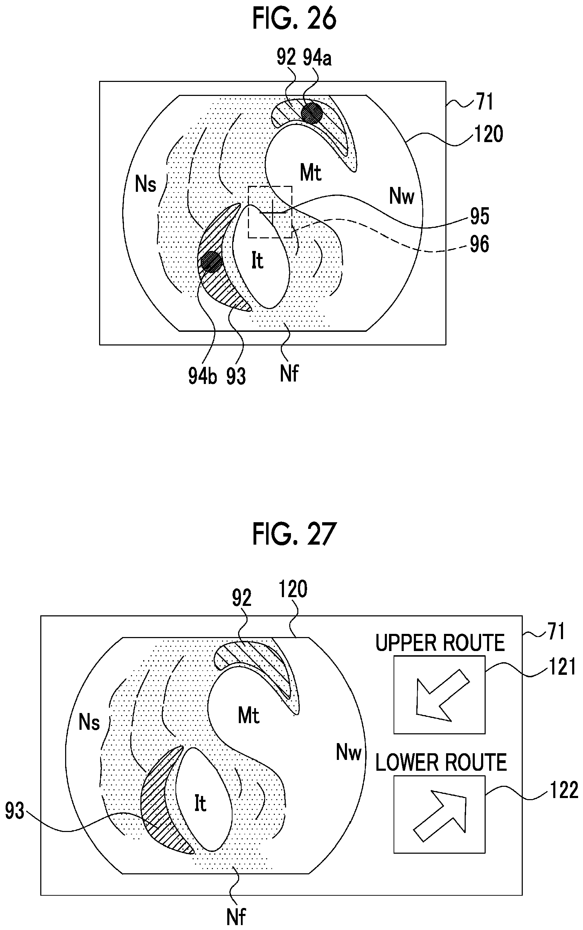

An example of a notification of the observation support information in a case in which the anterior nasal image is input to the nasal cavity operation support algorithm will be described. In a case in which the insertion route information is notified as the observation support information, the notification controller 70 generates the guide image 71 as illustrated in Fig. 26. In the example of the guide image 71 shown in Fig. 26, an insertion route guide mark 94a indicating the coordinates of the center position of the upper route insertion region 92, an insertion route guide mark 94b indicating the coordinates of the center position of the lower route insertion region 93, an image center guide mark 95 indicating the center position of the anterior nasal image 120, and a guide frame 96 are displayed. In this case, the user performs the operation of the endoscope 22 such that a position of the insertion route guide mark 94a or the insertion route guide mark 94b is aligned with the image center guide mark 95, whereby the distal end part 22d can be set to an ideal insertion position.

In addition, in a case in which the endoscope operation information is notified as the observation support information, the notification controller 70 generates the guide image 71 as illustrated in Fig. 27. In the example of the guide image 71 shown in Fig. 27, the detected upper route insertion region 92 and lower route insertion region 93 are superimposed and displayed on the anterior nasal image 120. An upper route operation display field 121 and a lower route operation display field 122 are displayed outside the anterior nasal image 120. In the example shown in Fig. 27, in the upper route operation display field 121, the moving direction of the distal end part 22d in the "lower left direction" is displayed as the endoscope operation information with respect to the upper route insertion region 92. In the lower route operation display field 122, the moving direction of the distal end part 22d in the "upper right direction" is displayed as the endoscope operation information with respect to the lower route insertion region 93.

In this case, a display mode of the upper route insertion region and the lower route insertion region may be changed according to the insertion route information output by the nasal cavity operation support algorithm. Specifically, a first threshold value for displaying an insertion region and a second threshold value for displaying an insertion region are provided with respect to the area of the upper route insertion region, and the display mode is switched to blue, yellow, and red in descending order of the area of the upper route insertion region. Similarly, a first threshold value for displaying an insertion region and a second threshold value for displaying an insertion region are provided with respect to the area of the lower route insertion region, and the display mode is switched to blue, yellow, and red in descending order of the area of the upper route insertion region. In the example shown in Fig. 27, the different display colors of the lower route insertion region 93 and the upper route insertion region 92 are represented by different hatches (diagonal lines). With the above configuration, it is possible to prompt the operator who is inexperienced in the transnasal insertion of the endoscope to select an insertion route along which the endoscope can be easily inserted.

Hereinafter, observation support information output by the nasopharyngeal operation support algorithm and a notification of the observation support information will be described. The nasopharyngeal operation support algorithm is an operation support algorithm for performing the operation support by indicating whether or not the distal end part 22d is in an appropriate observation position in the nasopharynx and by indicating the operation of the endoscope 22 such that the distal end part 22d is in an appropriate observation position.

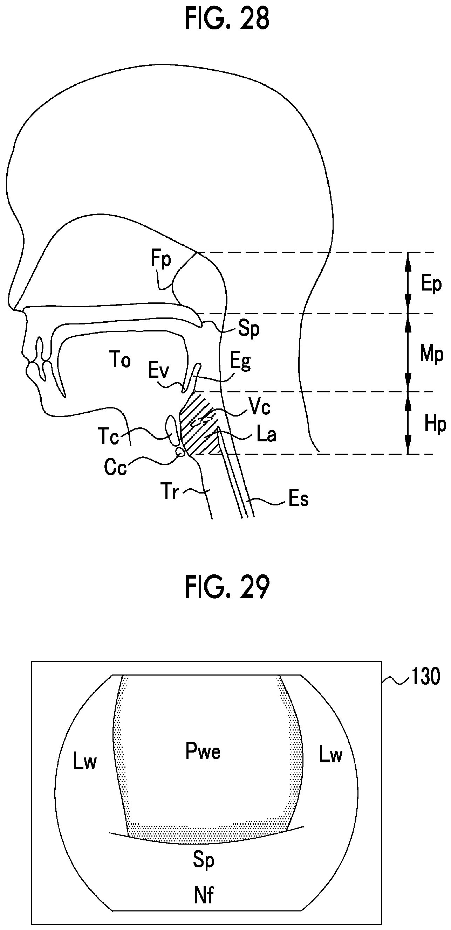

The nasopharynx is a region from the posterior nostril and a fornix pharyngis Fp to a root of a uvula. In terms of the height, as shown in Fig. 28, the nasopharynx Ep extends from a base of a skull to a transition part between the hard palate and the soft palate Sp, the oropharynx Mp extends from the transition part between the hard palate and the soft palate Sp to a bottom of an epiglottic vallecula Ev, and the hypopharynx Hp extends from the bottom of the epiglottic vallecula Ev to an inferior edge of a cricoid cartilage Cc. The larynx La is a region shown by a diagonal line, which is located at a height of the inferior edge of the cricoid cartilage Cc from a lingual surface of the epiglottis Eg and surrounded by the epiglottis Eg, a thyroid cartilage Tc, and left and right arytenoids. In a case in which the larynx is observed with the endoscope 22, the glottis Vc shown by a dotted line is recognized in Fig. 28.

Fig. 29 shows an example of a medical image in which the observation target is the nasopharynx (hereinafter, referred to as a nasopharyngeal image). The example shown in Fig. 29 is an example of a nasopharyngeal image 130 in which the distal end part 22d is disposed at an appropriate observation position (see (A) of Fig. 9). The appropriate observation position in the nasopharynx is a position where a posterior wall Pwe of the nasopharynx, left and right lateral walls Lw, the nasal floor Nf which is an inferior wall, and the soft palate Sp can be observed. The nasal floor Nf and the soft palate Sp are continuous, and, during the insertion or removal of the endoscope 22, the nasal floor Nf is observed on the front side (nasal side) and the soft palate Sp is observed on the back side (pharyngeal side). In this position, the movement of the soft palate during vocalization and swallowing can be observed, so that it is possible to evaluate an epipharyngeal closure function during vocalization and swallowing. In the orientation of the example of the nasopharyngeal image 130 shown in Fig. 29, the upper side of the paper is the head side, the lower side of the paper is the tail side, the front side of the paper is the ventral side, and the back side of the paper is the dorsal side.

The observation target identification algorithm as a trained model is trained using the nasopharyngeal image as shown in Fig. 29 as a learning image of "nasopharynx".

Hereinafter, a case in which the nasopharyngeal image is input to the nasopharyngeal operation support algorithm will be described. First, in a case in which the nasopharyngeal image as shown in Fig. 29 is input, the nasopharyngeal operation support algorithm outputs nasopharyngeal position information indicating that the position of the distal end part 22d is in an appropriate position or that the position of the distal end part 22d is in an inappropriate direction position such as "inappropriate right position", "inappropriate left position", "inappropriate upper position", "inappropriate lower position", "inappropriate back position", and "inappropriate front position".

The "inappropriate right position" refers to the nasopharyngeal position information in which the position of the distal end part 22d is too close to the right. Similarly, the nasopharyngeal position information of "inappropriate left position" is output in a case in which the position of the distal end part 22d is too close to the left, the nasopharyngeal position information of "inappropriate upper position" is output in a case in which the position of the distal end part 22d is too close to the top, the nasopharyngeal position information of "inappropriate lower position" is output in a case in which the position of the distal end part 22d is too close to the bottom, the nasopharyngeal position information of "inappropriate back position" is output in a case in which the position of the distal end part 22d is too close to the back side (pharyngeal side), and the nasopharyngeal position information of "inappropriate front position" is output in a case in which the position of the distal end part 22d is too close to the front side (nasal side). The inappropriate direction position is not limited to this, and the nasopharyngeal operation support algorithm outputs multi-classified nasopharyngeal position information such as "inappropriate lower left position" and "inappropriate upper right back position". In addition, the nasopharyngeal operation support algorithm may output a plurality of nasopharyngeal position information such that the nasopharyngeal position information of "inappropriate left position" and the nasopharyngeal position information of "inappropriate back position" are output.

It is preferable that the nasopharyngeal operation support algorithm that outputs the nasopharyngeal position information is a trained model that has been trained using a learning image including the nasopharyngeal image associated with the nasopharyngeal position information. As a learning model used to generate the nasopharyngeal operation support algorithm as the trained model, a convolutional neural network such as VGGNet, such as VGG16 or VGG19, or ResNet is suitable. The nasopharyngeal operation support algorithm may be an algorithm that outputs the nasopharyngeal position information by pattern matching with a learning image.

Next, the nasopharyngeal operation support algorithm outputs endoscope operation information based on the nasopharyngeal position information. In a case in which the nasopharyngeal position information is "in an appropriate position", the nasopharyngeal operation support algorithm outputs the endoscope operation information of "makes the endoscope 22 stationary". On the other hand, in a case in which the nasopharyngeal position information is "in an inappropriate direction position", the nasopharyngeal operation support algorithm outputs the moving direction of the distal end part 22d for moving the distal end part 22d in a direction opposite to the inappropriate direction indicated by the inappropriate direction position, as the endoscope operation information.

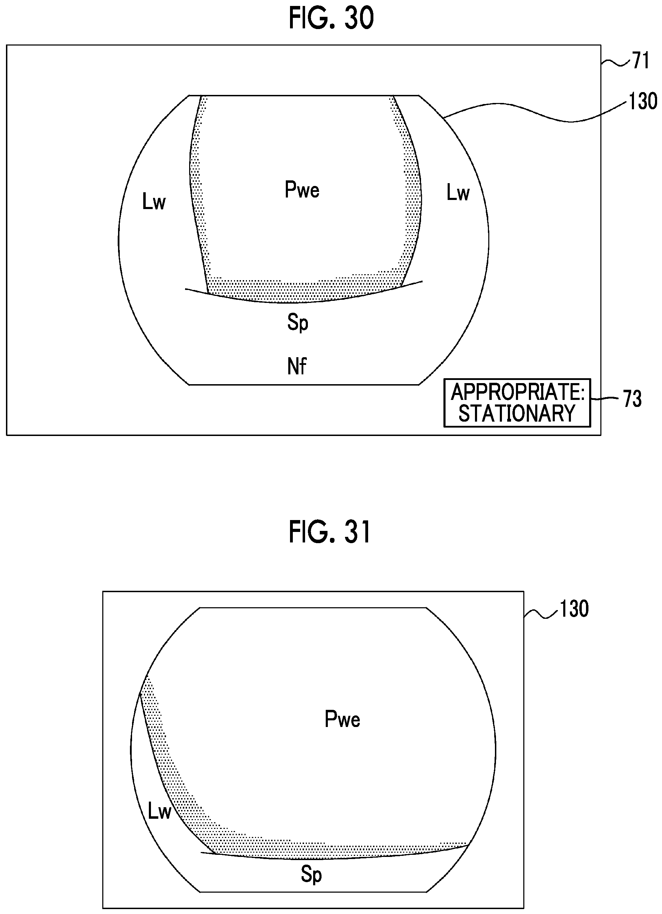

An example of a notification of the endoscope operation information output by the nasopharyngeal operation support algorithm will be described. For example, in a case in which the nasopharyngeal image 130 as shown in Fig. 29 is input to the nasopharyngeal operation support algorithm, the notification controller 70 generates the guide image 71 as shown in Fig. 30. In the example of the guide image 71 shown in Fig. 30, the observation support information display field 73 is provided at a position different from that of the nasopharyngeal image 130, and a message of "appropriate: stationary" is displayed in the observation support information display field 73. In this case, "in an appropriate position" is output as the nasopharyngeal position information.

In addition, instead of a message, a mark indicating "in an appropriate position" that instructs stop of the distal end part 22d may be displayed. For example, in a case of "in an appropriate position", a green frame is displayed around the nasopharyngeal image 130. By displaying such a guide image 71, the operator can confirm that the distal end part 22d is in an appropriate observation position. In addition, in a case in which the nasopharyngeal position information of "in an appropriate position" is output, the notification controller 70 may generate a voice message of "it is an appropriate position, please keep the endoscope stationary" from the speaker.

Hereinafter, an example of a notification of the endoscope operation information in a case of "in an inappropriate direction position" will be described. For example, the nasopharyngeal image 130 as shown in Fig. 31 is input to the nasopharyngeal operation support algorithm. The nasopharyngeal image 130 shown in Fig. 31 is a nasopharyngeal image of "inappropriate back position" in which the posterior wall Pwe of the nasopharynx appears large, the lateral wall Lw is slightly visible on the left side of the nasopharyngeal image 130, the soft palate Sp is slightly visible on the lower side, the lateral wall on the right side (that is, on the left side of the subject) is not visible, and the distal end part 22d is too close to the back side. In such a case, an operation of pulling out the distal end part 22d forward is required. In a case in which the nasopharyngeal image illustrated in Fig. 31 is input to the nasopharyngeal operation support algorithm, "inappropriate back position" is output as the nasopharyngeal position information.

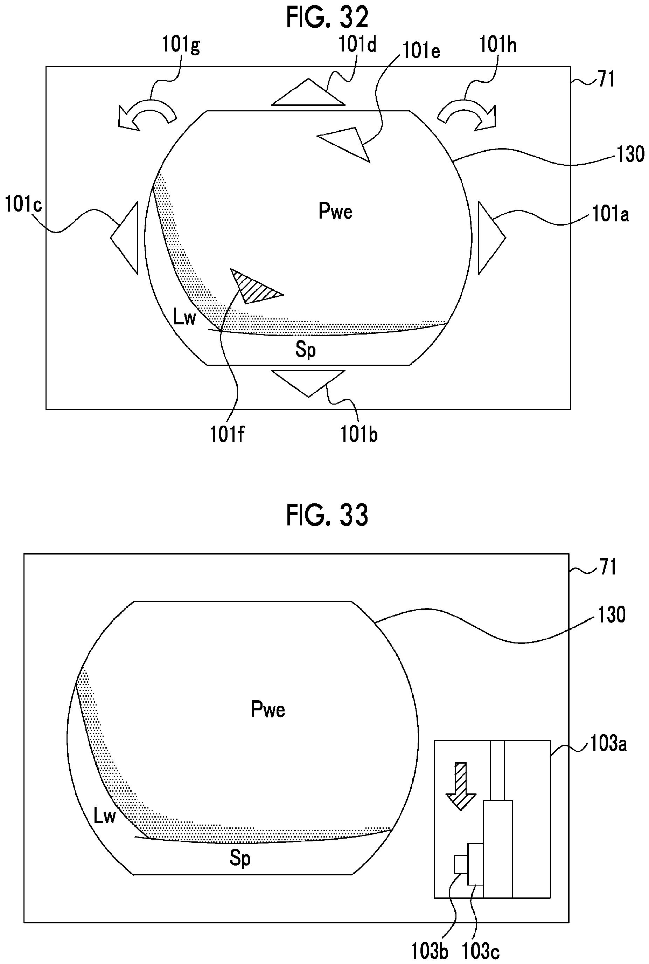

In this case, the notification controller 70 generates the guide image 71 as shown in Fig. 32. In the example of the guide image 71 shown in Fig. 32, guide direction display icons 101a, 101b, 101c, and 101d indicating the upward, downward, left, and right directions are displayed around the nasopharyngeal image 130. In addition, a guide direction display icon 101e indicating the backward direction and a guide direction display icon 101f indicating the forward direction are superimposed and displayed on the nasopharyngeal image 130. Further, the guide image 71 displays a guide direction display icon 101g indicating the left turn and a guide direction display icon 101h indicating the right turn. In the guide image 71 illustrated in Fig. 32, the difference in display mode (color) between the guide direction display icon 101f indicating the forward direction, and the other guide direction display icons 101a, 101b, 101c, 101d, 101e, 101g, and 101h is represented by the presence or absence of a diagonal line, which indicates that the endoscope 22 is operated in the forward direction.

The display mode of the guide direction display icon is not limited to this. For example, all of the guide direction display icons in the upward direction, downward direction, left direction, right direction, backward direction, forward direction, right turn, and left turn may be displayed on the guide image, and a part thereof may be displayed on the guide image. In addition, a message such as "too close to the back: pulling the endoscope" may be displayed on the guide image 71.

In addition, as the endoscope operation information, the endoscope operation support diagram 103a showing an operation for pulling out the endoscope 22 in the forward direction, as shown in Fig. 33, may be displayed on the guide image 71. A voice message of "please pull the endoscope" may be emitted from the speaker as the voice instruction information.

With the above configuration, it is possible to support the inexperienced operator in observing the nasopharynx by indicating whether the endoscope is in an appropriate observation position of the nasopharynx and, in a case in which the endoscope is in an inappropriate observation position, by indicating the operation of the endoscope. As a result, the burden on the subject can be reduced.

Hereinafter, a function of the oropharyngeal position determination algorithm, observation support information output by the oropharyngeal operation support algorithm, and a notification of the observation support information will be described. The oropharyngeal position determination algorithm is a position determination algorithm for glottis distant view observation that indicates whether or not the distal end part 22d is in an appropriate observation position in the oropharynx. The oropharyngeal operation support algorithm is an operation support algorithm that performs the operation support by indicating the operation of the endoscope 22 such that the distal end part 22d is in an appropriate observation position, based on an output result of the oropharyngeal position determination algorithm.

An example of the oropharyngeal image is shown in Fig. 34. Fig. 34 is an example of the medical image 72 displayed on the guide image 71 illustrated in Fig. 5. The oropharyngeal image 140 shown in Fig. 34 is a medical image in which the distal end part 22d is disposed at an appropriate observation position. The appropriate observation position in the oropharynx is a position where the posterior pharyngeal wall Pw, the tongue root Rl, and the entire larynx can be observed. As a specific configuration of the larynx, the rima glottidis Rg, the vocal fold Vof, a vestibular fold Vef (also referred to as a false vocal cord), the epiglottis Eg, the pyriform sinus Ps, and the like can be observed. The rima glottidis Rg is a space between the left and right vocal folds Vof. Further, in a case of performing the near view observation, an aryepiglottic fold Af, a cuneiform tubercle Cut, and a corniculate tubercle Cot, which will be described below, can be clearly observed on the outer side of the vestibular fold Vef. In this position, a left-right difference in movement of a pharyngeal wall during breathing, vocalization, and swallowing can be observed in a distant view, and the presence or absence of an abnormality can be evaluated. In addition, in common with the observation in the hypopharynx, the presence or absence of accumulation of saliva, residual food, or the like can be evaluated.

On the other hand, the oropharyngeal image 140 shown in Fig. 35 is a medical image in which the position of the distal end part 22d is inappropriate. Fig. 35 is an example of the medical image 72 displayed on the guide image 71 illustrated in Fig. 6. The oropharyngeal image 140 shown in Fig. 35 is an image captured too close to the larynx. The oropharyngeal operation support algorithm determines whether or not the image is captured too close to the larynx.

In the orientation of the example of the oropharyngeal image 140 shown in Figs. 34 and 35, the upper side of the paper is the dorsal side, the lower side of the paper is the ventral side, the front side of the paper is the head side, and the back side of the paper is the tail side. The observation target identification algorithm as a trained model is trained using the oropharyngeal image as shown in Figs. 34 and 35 as a learning image of "oropharynx".

Hereinafter, a flow of processing for outputting the observation support information in a case in which the oropharyngeal image is input to the oropharyngeal observation support algorithm (the oropharyngeal position determination algorithm and the oropharyngeal operation support algorithm) will be described. First, the oropharyngeal position determination algorithm to which the oropharyngeal image as shown in Fig. 34 or Fig. 35 is input outputs the endoscope position determination information indicating that the position of the distal end part 22d is "appropriate" or "inappropriate".

In a case in which the oropharyngeal image as shown in Fig. 34 is input, the oropharyngeal position determination algorithm outputs that the position of the distal end part 22d is "appropriate" as the endoscope position determination information. On the other hand, in a case in which the oropharyngeal image as shown in Fig. 35 is input, the oropharyngeal position determination algorithm outputs that the position of the distal end part 22d is "inappropriate" as the endoscope position determination information.

It is preferable that the oropharyngeal position determination algorithm that outputs the endoscope position determination information is a trained model that has been trained using a learning image including the oropharyngeal image associated with the endoscope position determination information indicating that the position of the distal end part 22d is "appropriate" or "inappropriate". In a case in which the supervised learning or the semi-supervised learning is applied to the learning model, the oropharyngeal image as shown in Fig. 34 may be a ground truth image for "appropriate". As a learning model used to generate the oropharyngeal operation support algorithm as the trained model, a convolutional neural network such as VGGNet, such as VGG16 or VGG19, or ResNet is suitable. The oropharyngeal operation support algorithm may be an algorithm that outputs the endoscope position determination information by pattern matching with a learning image.

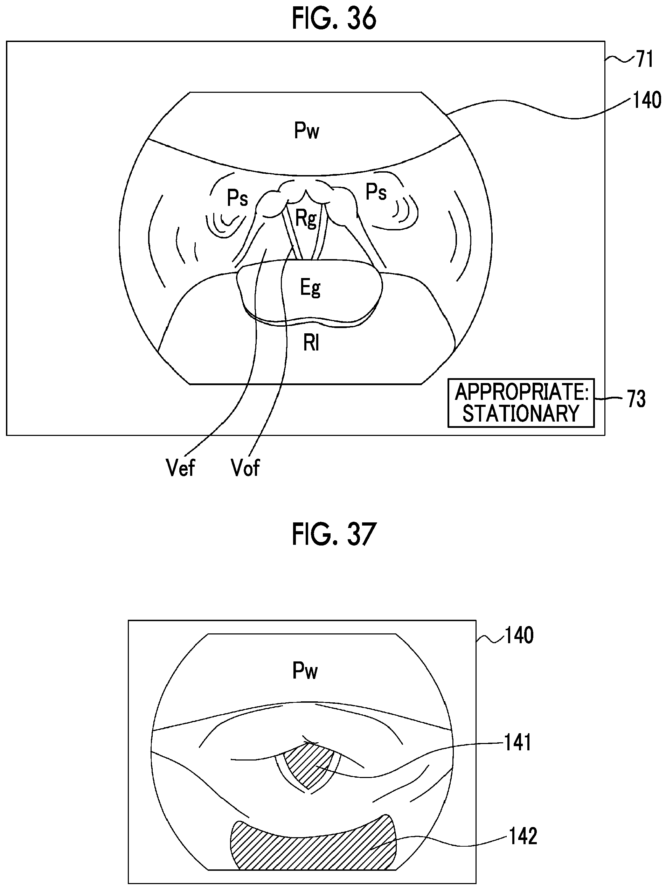

Next, the oropharyngeal operation support algorithm outputs endoscope operation information based on the endoscope position determination information. In a case in which the endoscope position determination information is "appropriate", the oropharyngeal operation support algorithm outputs the endoscope operation information of "makes the endoscope 22 stationary". In this case, the notification controller 70 generates the guide image 71 as shown in Fig. 36. In the example of the guide image 71 shown in Fig. 36, the observation support information display field 73 is provided at a position different from that of the oropharyngeal image 140, and a message of "appropriate: stationary" indicating the observation position is appropriate is displayed in the observation support information display field 73. In addition, instead of a message, a mark indicating "appropriate" for providing an instruction to make the distal end part 22d stationary may be displayed. For example, in a case of "appropriate", a green frame is displayed around the oropharyngeal image 140. In this case, the notification controller 70 performs a control of switching the display of the endoscope operation information such that, in a case of "inappropriate", the mark indicating "appropriate" in the guide image 71 is hidden and the guide direction display icon or the like is displayed.

In addition, in a case in which the endoscope position determination information of "appropriate" is output, the notification controller 70 may generate a voice message of "it is an appropriate position, please keep the endoscope stationary" from the speaker. By performing the notification by such a guide image or voice, the operator can confirm that the distal end part 22d is in an appropriate observation position of the oropharynx.

Hereinafter, a method of outputting the endoscope operation information by the oropharyngeal operation support algorithm and an example of a notification of the endoscope operation information in a case in which the endoscope position determination information is "inappropriate" will be described. It is preferable that the latest medical image is input to the oropharyngeal operation support algorithm (see Fig. 4). The same medical image as the medical image input to the oropharyngeal position determination algorithm may be input to the oropharyngeal operation support algorithm.

First, the oropharyngeal operation support algorithm outputs oropharyngeal region information. Specifically, the oropharyngeal operation support algorithm to which the oropharyngeal image 140 as shown in Fig. 35 is input outputs a glottis region 141 and an epiglottis region 142 as the oropharyngeal region information, as shown in Fig. 37. As shown in Fig. 37, the glottis region may be a region of the rima glottidis Rg during breathing, or may be a region of the rima glottidis Rg during vocalization, a region including the left and right vocal folds Vof and the rima glottidis Rg, and a region of the left and right vocal folds Vof not including the rima glottidis Rg.

It is preferable that the oropharyngeal operation support algorithm that outputs the oropharyngeal region information is a trained model that outputs the glottis region and the epiglottis region by segmentation on the medical image. The trained model is generated by being trained using the oropharyngeal image in which the glottis region and the epiglottis region are classified in advance by the doctor as a learning image which is a ground truth image. As a learning model applied to the generation of such a trained model, it is preferable to apply PSPnet, and other learning models suitable for segmentation, such as SPPnet and Segnet, may be used. The oropharyngeal operation support algorithm may be a trained model generated by training a learning model to which unsupervised learning is applied.

Next, the oropharyngeal operation support algorithm outputs oropharyngeal region arithmetic information using the oropharyngeal region information. The oropharyngeal region arithmetic information is information used for outputting the endoscope operation information such that the position of the distal end part 22d is an appropriate observation position. The appropriate observation position is information added to an "appropriate" oropharyngeal image in a case of being used as a learning image.

The oropharyngeal region arithmetic information is the area, the width, and/or the coordinate information of the center position of the glottis region and the epiglottis region. The areas of the glottis region and the epiglottis region is calculated by a method of calculating the areas of the regions classified as these regions by segmentation. The width of the glottis region or the width of the epiglottis region refers to a distance between any two points of pixels constituting the glottis region or the epiglottis region. The width of the glottis region may be a distance between the left and right vocal folds Vof, or may be a distance between a point most on the dorsal side and a point most on the ventral side in the region of the rima glottidis Rg. The coordinate information of the center positions of the glottis region and the epiglottis region is the center of gravity or the geometric center of these regions. A calculation method of the center positions of the glottis region and the epiglottis region is the same as the calculation method of the center position of the upper route insertion region in the upper route image of the nasal cavity, and thus the description thereof will be omitted.

The oropharyngeal operation support algorithm outputs endoscope operation information using the oropharyngeal region arithmetic information. Specifically, a first threshold value for glottis distant view observation is provided with respect to the area of the glottis region or the epiglottis region, and, in a case in which the area of the glottis region or the epiglottis region is larger than the first threshold value for glottis distant view observation, endoscope operation instruction information of "forward direction" is output as the moving direction of the distal end part 22d. In addition, a second threshold value for glottis distant view observation is provided with respect to the area of the glottis region or the epiglottis region, and, in a case in which the area of the glottis region or the epiglottis region is less than the second threshold value for glottis distant view observation, endoscope operation instruction information of "backward direction" is output as the moving direction of the distal end part 22d. The first threshold value for glottis distant view observation and the second threshold value for glottis distant view observation are values that can be optionally set, and may be values respectively set for the areas of the glottis region and the epiglottis region, or values given to either one of them. The width of the glottis region or the epiglottis region may be used instead of the area of the glottis region or the epiglottis region, or the endoscope operation information may be output based on a combination thereof.

In addition, the center positions of the glottis region and the epiglottis region output using the oropharyngeal image input to the oropharyngeal operation support algorithm may be compared with the center positions of the glottis region and the epiglottis region in the oropharyngeal image in which the position of the distal end part 22d is "appropriate", and the endoscope operation information may be output based on a difference thereof. In this case, for example, a difference between the center positions of the glottis region and the epiglottis region in the oropharyngeal image in which the position of the distal end part 22d is "inappropriate" and the center positions of the glottis region and the epiglottis region in the oropharyngeal image in which the position of the distal end part 22d is "appropriate" can be calculated as a vector indicating the moving direction and the moving amount of the distal end part 22d of the endoscope 22, and can be used as the endoscope operation information.

In addition, a current position vector may be generated by connecting the center position of the glottis region and the center position of the epiglottis region output using the oropharyngeal image input to the oropharyngeal operation support algorithm, and an appropriate position vector may be generated by connecting the center position of the glottis region and the center position of the epiglottis region in the oropharyngeal image in which the position of the distal end part 22d is "appropriate", and the moving direction, the moving distance, and the rotation angle of the distal end part 22d may be output as the endoscope operation instruction information based on a difference between the current position vector and the appropriate position vector.

Hereinafter, an example of a notification of the endoscope operation information in a case in which the endoscope position determination information is "inappropriate" will be described. For example, it is assumed that the oropharyngeal operation support algorithm to which the oropharyngeal image 140 as shown in Fig. 35 is input outputs the endoscope operation instruction information of "backward direction" as the moving direction of the distal end part 22d.

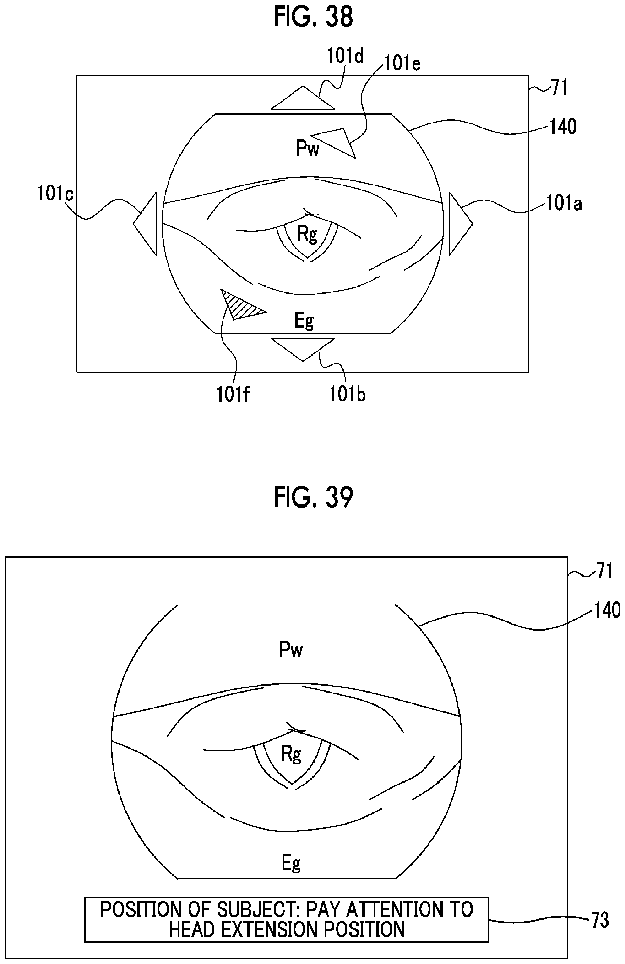

In this case, the notification controller 70 generates the guide image 71 as shown in Fig. 38. In the example of the guide image 71 shown in Fig. 38, guide direction display icons 101a, 101b, 101c, and 101d indicating the upward, downward, left, and right directions are displayed around the oropharyngeal image 140. In addition, guide direction display icons 101e and 101f indicating the backward direction and the forward direction are superimposed and displayed on the oropharyngeal image 140. In the guide image 71 illustrated in Fig. 38, the difference in display mode (color) between the guide direction display icon 101f indicating the forward direction, and the other guide direction display icons 101a, 101b, 101c, 101d, and 101e is represented by the presence or absence of a diagonal line, which indicates that the endoscope 22 is operated in the forward direction. The display mode of the guide direction display icon is not limited to this. For example, the guide image 71 may display a guide direction display icon 101g indicating the left turn and a guide direction display icon 101h indicating the right turn, as shown in Fig. 32.

In addition, the guide image 71 may display an endoscope operation support diagram showing the operation of the angle knob 22e for bending and moving the distal end part 22d or the pulling-out or insertion direction of the endoscope 22, as the endoscope operation information (see Fig. 33 and the like). Since it is the same as the example of the nasopharyngeal image 130, the illustration is omitted. With the above configuration, it is possible to support the inexperienced operator in observing the oropharynx by indicating whether the endoscope is in an appropriate observation position of the oropharynx and, in a case in which the endoscope is in an inappropriate observation position, by indicating the operation of the endoscope. As a result, the burden on the subject can be reduced.

In addition, the oropharyngeal operation support algorithm outputs subject position information using the oropharyngeal region arithmetic information. For example, in a case in which the oropharyngeal image 140 as shown in Fig. 35 is input, the oropharyngeal operation support algorithm outputs the subject position information of "head is bent forward, check for head extension". In this case, the notification controller 70 generates the guide image 71 as shown in Fig. 39. In the example of the guide image 71 shown in Fig. 39, a message "position of subject: pay attention to head extension position" for prompting the subject to change the position is displayed in the observation support information display field 73. In addition, as the voice instruction information, a voice message of "head may be bent forward, please pay attention to head extension position" may be emitted from the speaker.

It is preferable that the oropharyngeal operation support algorithm that outputs the subject position information is a trained model that has been trained using a learning image in which information related to the posture of the subject, such as "head is bent forward", is added to the oropharyngeal image 140 as shown in Fig. 35. The oropharyngeal operation support algorithm that outputs the endoscope operation information and the oropharyngeal operation support algorithm that outputs the subject position information may be the same trained model or different trained models. In a case in which these are different trained models, a first oropharyngeal operation support algorithm that outputs the endoscope operation information and a second oropharyngeal operation support algorithm that outputs the subject position information are provided, and the latest oropharyngeal image is input to each of the first oropharyngeal operation support algorithm and the second oropharyngeal operation support algorithm.

Hereinafter, a function of the hypopharyngeal position determination algorithm, observation support information output by the hypopharyngeal operation support algorithm, and a notification of the observation support information will be described. The hypopharyngeal position determination algorithm is a position determination algorithm for glottis near view observation that indicates whether or not the distal end part 22d is in an appropriate observation position in the hypopharynx. The hypopharyngeal operation support algorithm is an operation support algorithm that performs the operation support by indicating the operation of the endoscope 22 such that the distal end part 22d is in an appropriate observation position, based on an output result of the hypopharyngeal position determination algorithm.

Fig. 40 shows an example of a medical image in which the observation target is the hypopharynx (hereinafter, referred to as a hypopharyngeal image). A hypopharyngeal image 150 shown in Fig. 40 is a hypopharyngeal image in which the distal end part 22d is disposed at an appropriate observation position. The appropriate observation position in the hypopharynx is a position where the entire arytenoid part can be observed. Specifically, it is a position where the rima glottidis Rg, the left and right vocal folds Vof, the left and right vestibular folds Vef, the left and right aryepiglottic folds Af, the left and right cuneiform tubercles Cut, the left and right corniculate tubercles Cot, the left and right pyriform sinuses Ps, and the like can be clearly observed. In this position, tracheal surfaces of the posterior pharyngeal wall Pw and the epiglottis Eg are observed on the upper and lower sides of the hypopharyngeal image. In this position, the arytenoid part can be observed in a near view during breathing, vocalization, and swallowing, and the movement of the arytenoid part, the presence or absence of an organic abnormality, and the presence or absence of accumulation of saliva, residual food, or the like can be evaluated.

On the other hand, the hypopharyngeal image 150 shown in Fig. 41 is an example of the hypopharyngeal image 150 in which the position of the distal end part 22d is inappropriate. The hypopharyngeal image 150 shown in Fig. 41 is an image in which the entire rima glottidis Rg and left and right vocal folds Vof are not recognized, and the entire arytenoid part cannot be clearly observed. In the orientation of the example of the oropharyngeal image 140 shown in Figs. 40 and 41, the upper side of the paper is the dorsal side, the lower side of the paper is the ventral side, the front side of the paper is the head side, and the back side of the paper is the tail side.

The observation target identification algorithm as a trained model is trained using the hypopharyngeal image as shown in Figs. 40 and 41 as a learning image of "hypopharynx".

Hereinafter, a flow of processing for outputting the observation support information in a case in which the hypopharyngeal image is input to the hypopharyngeal observation support algorithm (the hypopharyngeal position determination algorithm and the hypopharyngeal operation support algorithm) will be described. First, the hypopharyngeal position determination algorithm to which the hypopharyngeal image as shown in Figs. 40 and 41 is input outputs the endoscope position determination information indicating that the position of the distal end part 22d is "appropriate" or "inappropriate".

In a case in which the hypopharyngeal image as shown in Fig. 40 is input, the hypopharyngeal position determination algorithm outputs that the position of the distal end part 22d is "appropriate" as the endoscope position determination information. On the other hand, in a case in which the hypopharyngeal image as shown in Fig. 41 is input, the hypopharyngeal position determination algorithm outputs that the position of the distal end part 22d is "inappropriate" as the endoscope position determination information.

It is preferable that the hypopharyngeal position determination algorithm that outputs the endoscope position determination information is a trained model that has been trained using a learning image including the hypopharyngeal image associated with the endoscope position determination information indicating that the position of the distal end part 22d is "appropriate" or "inappropriate". In a case in which the supervised learning or the semi-supervised learning is applied to the learning model, the hypopharyngeal image as shown in Fig. 40 may be a ground truth image for "appropriate". As a learning model used to generate the hypopharyngeal operation support algorithm as the trained model, a convolutional neural network such as VGGNet, such as VGG16 or VGG19, or ResNet is suitable. The hypopharyngeal operation support algorithm may be an algorithm that outputs the endoscope position determination information by pattern matching with a learning image.

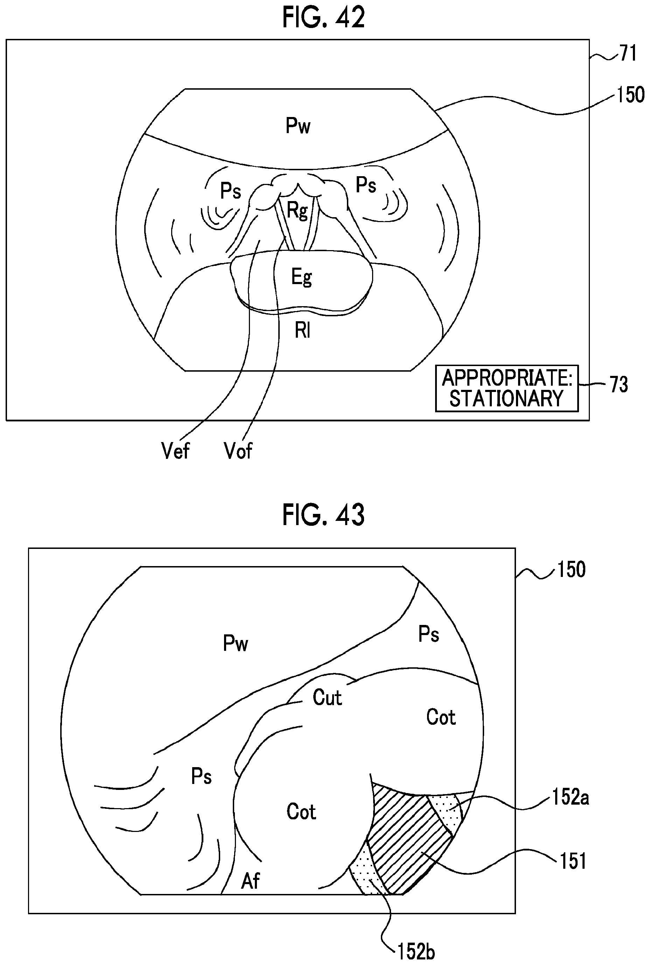

Next, the hypopharyngeal operation support algorithm outputs endoscope operation information based on the endoscope position determination information. In a case in which the endoscope position determination information is "appropriate", the hypopharyngeal operation support algorithm outputs the endoscope operation information of "makes the endoscope 22 stationary". In this case, the notification controller 70 generates the guide image 71 as shown in Fig. 42. In the example of the guide image 71 shown in Fig. 42, a message of "appropriate: stationary" indicating the observation position is appropriate is displayed in the observation support information display field 73 provided at a position different from that of the hypopharyngeal image 150. In addition, instead of a message, a mark indicating "appropriate" for providing an instruction to make the distal end part 22d stationary may be displayed. For example, in a case of "appropriate", a green frame is displayed around the hypopharyngeal image 150.

In addition, in a case in which the endoscope position determination information of "appropriate" is output, the notification controller 70 may generate a voice message of "it is an appropriate position, please keep the endoscope stationary" from the speaker. By performing the notification by such a guide image or voice, the operator can confirm that the distal end part 22d is in an appropriate observation position of the hypopharynx.

Hereinafter, a method of outputting the endoscope operation information by the hypopharyngeal operation support algorithm and an example of a notification of the endoscope operation information in a case in which the endoscope position determination information is "inappropriate" will be described. It is preferable that the latest medical image is input to the hypopharyngeal operation support algorithm (see Fig. 4). The same medical image as the medical image input to the hypopharyngeal position determination algorithm may be input to the hypopharyngeal operation support algorithm.

First, the hypopharyngeal operation support algorithm outputs hypopharyngeal region information. The hypopharyngeal operation support algorithm to which the hypopharyngeal image 150 as shown in Fig. 41 is input outputs a glottis region 151 and left and right vocal fold regions 152a and 152b as the hypopharyngeal region information, as shown in Fig. 43. The glottis region 151 detected by the hypopharyngeal operation support algorithm is not limited to the example shown in Fig. 43, as in a case of the oropharynx.

It is preferable that the hypopharyngeal operation support algorithm that outputs the hypopharyngeal region information is a trained model that outputs the glottis region and the vocal fold region by segmentation on the medical image. The trained model is generated by being trained using the hypopharyngeal image in which the glottis region and the vocal fold region are classified in advance by the doctor as a learning image which is a ground truth image. The left and right vocal fold regions may be classified into different classes on the left and right sides, or may be classified into the same class.

As a learning model applied to the generation of such a trained model, it is preferable to apply PSPnet, and other learning models suitable for segmentation, such as SPPnet and Segnet, may be used. The hypopharyngeal operation support algorithm may be a trained model generated by training a learning model to which unsupervised learning is applied.

Next, the hypopharyngeal operation support algorithm outputs hypopharyngeal region arithmetic information using the hypopharyngeal region information. The hypopharyngeal region arithmetic information is information used for outputting the endoscope operation information such that the position of the distal end part 22d is an appropriate observation position. The appropriate observation position is information added to an "appropriate" hypopharyngeal image in a case of being used as a learning image.

The hypopharyngeal region arithmetic information is the area, the width, and/or the coordinate information of the center position of the glottis region, and the length of the vocal fold. The area of the glottis region is calculated by a method of calculating the areas of the regions classified as these regions by segmentation. The width of the glottis region refers to a distance between any two points included in pixels constituting the glottis region, and is not limited to a distance between the left and right vocal folds Vof, as with the width of the glottis region in the oropharynx.

The length of the vocal fold region is the largest of distances between any two points included in pixels constituting the vocal fold region. For example, it is a distance (length) between an end of the vocal fold on the dorsal side (esophageal side) and an end of the vocal fold on the ventral side (epiglottic side). In addition, the length of the vocal fold region may be calculated for the length of the left and right vocal fold regions, and the length of either vocal fold region may be output as the hypopharyngeal region arithmetic information, or the length of both vocal fold regions may be output as the hypopharyngeal region arithmetic information.

The coordinate information of the center position of the glottis region is the center of gravity or the geometric center of these regions. A calculation method of the center position of the glottis region is the same as the calculation method of the center position of the upper route insertion region in the upper route image of the nasal cavity, and thus the description thereof will be omitted.

The hypopharyngeal operation support algorithm outputs endoscope operation information using the hypopharyngeal region arithmetic information. Specifically, a first threshold value for glottis near view observation is provided with respect to the area of the glottis region, and, in a case in which the area of the glottis region is larger than the first threshold value for glottis near view observation, endoscope operation instruction information of "forward direction" is output as the moving direction of the distal end part 22d. In addition, a second threshold value for glottis near view observation is provided with respect to the area of the glottis region, and, in a case in which the area of the glottis region is less than the second threshold value for glottis near view observation, endoscope operation instruction information of "backward direction" is output as the moving direction of the distal end part 22d. The first threshold value for glottis near view observation and the second threshold value for glottis near view observation are values that can be optionally set, and may be values respectively set for the areas of the glottis region and the vocal fold region, or values given to either one of them. The width of the glottis region may be used instead of the area of the glottis region, or the endoscope operation information may be output based on a combination thereof.

In addition, a threshold value such as a first threshold value for glottis near view observation and a second threshold value for glottis near view observation may be provided with respect to the length of the vocal fold, and the endoscope operation instruction information may be output according to the length of the vocal fold.

In addition, the center position of the glottis region output using the hypopharyngeal image input to the hypopharyngeal operation support algorithm may be compared with the center position of the glottis region in the hypopharyngeal image in which the position of the distal end part 22d is "appropriate", and the endoscope operation information may be output based on a difference thereof. In this case, for example, a difference between the center position of the glottis region in the hypopharyngeal image in which the position of the distal end part 22d is "inappropriate" and the center position of the glottis region in the hypopharyngeal image in which the position of the distal end part 22d is "appropriate" can be calculated as a vector indicating the moving direction, the moving amount, and the rotation angle of the distal end part 22d of the endoscope 22, and can be used as the endoscope operation information.

Hereinafter, an example of a notification of the endoscope operation information in a case in which the endoscope position determination information is "inappropriate" will be described. For example, it is assumed that the hypopharyngeal operation support algorithm to which the hypopharyngeal image 150 as shown in Fig. 41 is input outputs the endoscope operation instruction information of "backward direction" as the moving direction of the distal end part 22d.

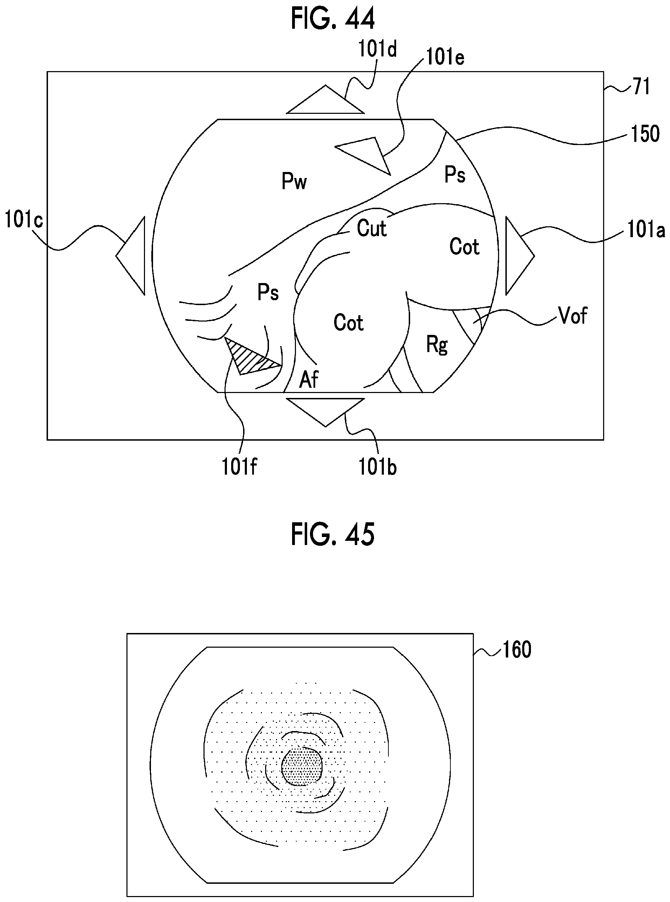

In this case, the notification controller 70 generates the guide image 71 as shown in Fig. 44. In the example of the guide image 71 shown in Fig. 44, guide direction display icons 101a, 101b, 101c, and 101d indicating the upward, downward, left, and right directions are displayed around the hypopharyngeal image 150. In addition, guide direction display icons 101e and 101f indicating the backward direction and the forward direction are superimposed and displayed on the hypopharyngeal image 150. In the guide image 71 illustrated in Fig. 44, the difference in display mode (color) between the guide direction display icon 101f indicating the forward direction, and the other guide direction display icons 101a, 101b, 101c, 101d, and 101e is represented by the presence or absence of a diagonal line, which indicates that the endoscope 22 is operated in the forward direction. The display mode of the guide direction display icon is not limited to this. For example, the guide image 71 may display a guide direction display icon 101g indicating the left turn and a guide direction display icon 101h indicating the right turn, as shown in Fig. 32.

In addition, the guide image 71 may display an endoscope operation support diagram showing the operation of the angle knob 22e for bending and moving the distal end part 22d or the pulling-out or insertion direction of the endoscope 22, as the endoscope operation information (see Fig. 33 and the like). With the above configuration, it is possible to support the inexperienced operator in observing the hypopharynx by indicating whether the endoscope is in an appropriate observation position of the hypopharynx and, in a case in which the endoscope is in an inappropriate observation position, by indicating the operation of the endoscope. As a result, the burden on the subject can be reduced.

In addition, the hypopharyngeal operation support algorithm outputs subject position information. Since a method of a notification of the subject position information is the same as the method of notifying of the subject position information in the example of the oropharynx, the description thereof will be omitted. It is preferable that the hypopharyngeal operation support algorithm is a trained model that has been trained using a learning image in which information related to the posture of the subject is added to the hypopharyngeal image 150. The hypopharyngeal operation support algorithm that outputs the endoscope operation information and the hypopharyngeal operation support algorithm that outputs the subject position information may be the same trained model or different trained models. In a case in which these are different trained models, a first hypopharyngeal operation support algorithm that outputs the endoscope operation information and a second hypopharyngeal operation support algorithm that outputs the subject position information are provided, and the latest hypopharyngeal image is input to each of the first hypopharyngeal operation support algorithm and the second hypopharyngeal operation support algorithm.

Hereinafter, observation support information output by the erroneous insertion operation support algorithm and a notification of the observation support information will be described. The erroneous insertion operation support algorithm is an operation support algorithm for performing the operation support by providing an operation instruction for pulling out the endoscope 22 in a case in which the endoscope 22 is erroneously inserted into the esophagus or the trachea (see Figs. 10 and 11).

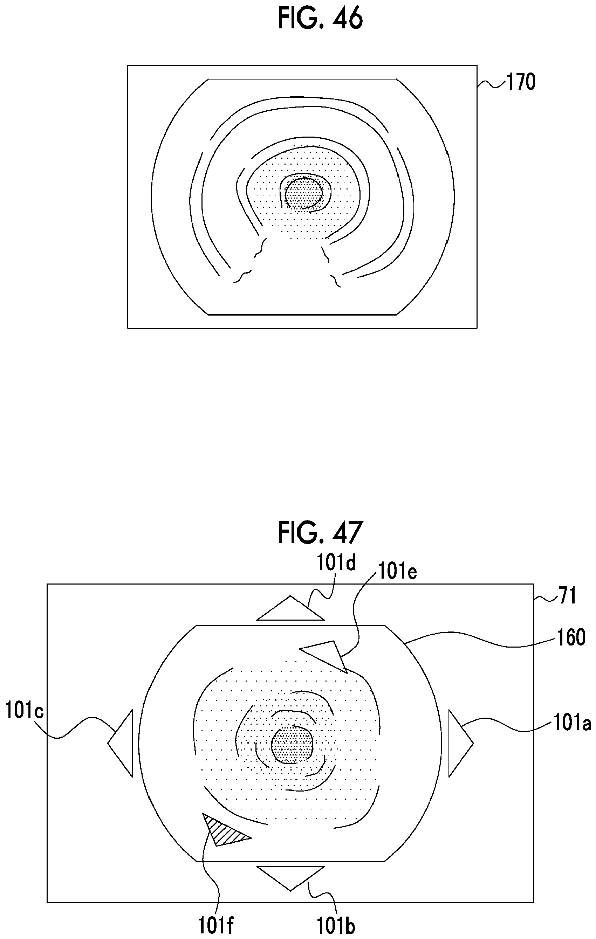

Fig. 45 shows an example of a medical image (esophageal image 160) in which the observation target is the esophagus in a case in which the endoscope 22 is erroneously inserted to the position shown in Fig. 10. In addition, Fig. 46 shows an example of a medical image (tracheal image 170) in which the observation target is the trachea in a case in which the endoscope 22 is erroneously inserted to the position shown in Fig. 11. The observation target identification algorithm as a trained model is trained using the esophageal image as shown in Fig. 45 as a learning image of "esophagus" or using the tracheal image as shown in Fig. 46 as a learning image of "trachea".

In a case in which the esophageal image as shown in Fig. 45 or the tracheal image as shown in Fig. 46 is input, the erroneous insertion operation support algorithm outputs endoscope operation information for providing an instruction to pull out the endoscope.

An example of a notification of the endoscope operation information in this case will be described. For example, in a case in which the esophageal image as shown in Fig. 45 is input to the erroneous insertion operation support algorithm, the notification controller 70 generates the guide image 71 as shown in Fig. 47. In the example of the guide image 71 shown in Fig. 47, guide direction display icons 101a, 101b, 101c, and 101d indicating the upward, downward, left, and right directions are displayed around the esophageal image 160. In addition, guide direction display icons 101e and 101f indicating the backward direction and the forward direction are superimposed and displayed on the oropharyngeal image 140. In the guide image 71 illustrated in Fig. 47, the difference in display mode (color) between the guide direction display icon 101f indicating the forward direction, and the other guide direction display icons 101a, 101b, 101c, 101d, and 101e is represented by the presence or absence of a diagonal line, which indicates that the endoscope 22 is operated in the forward direction. The display mode of the guide direction display icon is not limited to this.

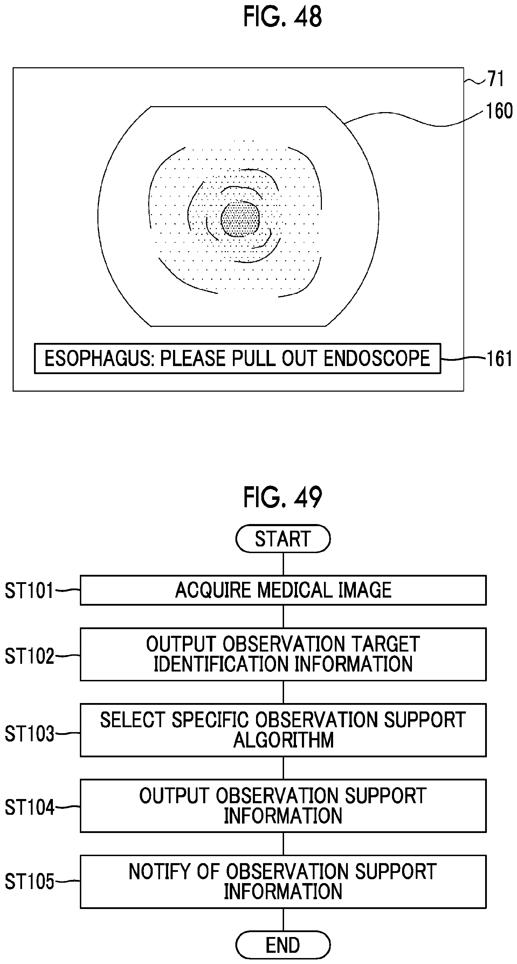

In addition, as in the example of the guide image 71 shown in Fig. 48, a warning display field 161 may be provided around the esophageal image 160 to perform a notification for prompting pulling-out of the endoscope 22 because the distal end part 22d has reached the esophagus. In the example shown in Fig. 48, a message of "esophagus: please pull out endoscope" is displayed. In addition, in a case in which the erroneous insertion operation support algorithm outputs the endoscope operation information for providing an instruction to pull out the endoscope, the notification controller 70 may perform a control of emitting a voice message of "please pull out endoscope" from the speaker as the voice instruction information. With the above configuration, it is possible to prompt the operator to pull out the endoscope in a case in which the image processing device 10 recognizes a part where the endoscope needs to be pulled out immediately.

Hereinafter, a notification of observation support information output by the operation support algorithm for out-of-observation-support-target will be described. In swallowing endoscopy, after the observation support target part is identified, the observation support target part may not be identified due to the inflow of a foreign substance such as food or saliva, or the contraction movement of the pharynx part caused by the swallowing movement or coughing. The observation support algorithm for out-of-observation-support-target outputs observation support stop information in such a case.

In a case in which the observation support stop information is output, the notification controller 70 temporarily hides the endoscope operation information that had been displayed in the guide image until immediately before. For example, the guide direction display icon that had been displayed until immediately before is hidden. In addition, the notification controller 70 may indicate that the operation instruction has been stopped by setting all the display modes of the guide direction display icons to be the same. In addition, a message of "CAD OFF" may be displayed on the guide image to notify the operator that the operation support is temporarily stopped (see Fig. 7).

In a case in which a medical image in which boundary regions between the nasal cavity and the nasopharynx, the nasopharynx and the oropharynx, and the oropharynx and the hypopharynx are the observation target is input to the observation target identification algorithm, "out of the observation support target" may be output. By excluding the boundary regions from the observation support target and narrowing a range in which the oropharyngeal observation support algorithm or the hypopharyngeal observation support algorithm is selected as the specific observation support algorithm, it is possible to prevent the execution of processing that causes inconsistency in the image processing, and to prevent the observation support that confuses the operator.

In addition, it is preferable to switch between the presence and absence of the notification of the observation support information. For example, in a case in which a guide image in which a guide direction display icon is displayed is displayed, a notification switching instruction may be transmitted to the notification controller 70 by operating the operating part 22b of the endoscope 22 or the foot switch, and the guide direction display icon of the guide image may be hidden. In addition, in a case in which the guide direction display icon of the guide image is hidden, the guide direction display icon may be displayed again by operating the operating part 22b of the endoscope 22 or the foot switch again. Further, a notification display switching button may be provided as a graphical user interface (GUI) on the guide image to switch between the presence and absence of the notification of the observation support information. With such a configuration of switching between the presence and absence of the notification of the observation support information, it is possible to perform the notification only in a case in which the operator needs the notification.

A series of flow of the operation method in the image processing device 10 according to the present embodiment will be described with reference to a flowchart of Fig. 49. First, the image acquisition unit 30 acquires a medical image from the modality 20 or the database 11 (step ST101). Next, the observation target identification unit 40 outputs observation target identification information by inputting the medical image to an observation target identification algorithm (step ST102). Next, the observation support algorithm selection unit 50 selects, from a plurality of observation support algorithms, one specific observation support algorithm based on the observation target identification information (step ST103). Next, the observation support information generation unit 60 outputs observation support information by inputting the medical image to the specific observation support algorithm (step ST104). Finally, the notification controller 70 performs a control of notifying of the observation support information, such as generating a guide image, voice instruction information, and the like (step ST105). The observation support information is notified via a user interface such as a display or a speaker.

In the above-described embodiment, in the image processing device 10, hardware structures of processing units executing various processes, such as the image acquisition unit 30, the observation target identification unit 40, the observation support algorithm selection unit 50, the observation support information generation unit 60, and the notification controller 70, are various processors as follows. The various processors include a central processing unit (CPU) that is a general-purpose processor that executes software (program) to function as various processing units, a programmable logic device (PLD) that is a processor of which a circuit configuration can be changed after manufacture, such as a field programmable gate array (FPGA), and an exclusive electric circuit that is a processor having a circuit configuration exclusively designed to execute various kinds of processing.

One processing unit may be configured of one of these various processors, or may be configured of a combination of two or more processors of the same type or different types (for example, a combination of a plurality of FPGAs or a combination of a CPU and an FPGA). In addition, a plurality of processing units may be configured of one processor. As an example in which the plurality of processing units are configured of one processor, first, as typified by a computer such as a client or a server, one processor is configured of a combination of one or more CPUs and software and this processor functions as the plurality of processing units. Second, as typified by a system on chip (SoC) or the like, a processor that realizes the functions of the entire system including the plurality of processing units by using one integrated circuit (IC) chip is used. As described above, the various processing units are configured using one or more of the various processors as a hardware structure.

Further, the hardware structure of these various processors is more specifically an electric circuit (circuitry) in a form in which circuit elements such as semiconductor elements are combined. The hardware structure of the storage unit is a storage device such as a hard disc drive (HDD) or a solid state drive (SSD).

### Explanation of References

10: image processing device
11: database
12: user interface
20: modality
21: endoscope system
22: endoscope
22a: insertion part
22b: operating part
22c: bendable part
22d: distal end part
22e: angle knob
22f: still image acquisition instruction switch
23: processor device
24: light source device
30: image acquisition unit
31, 32, 33, 72: medical image
40: observation target identification unit
41: observation target identification information
50: observation support algorithm selection unit
51: specific observation support algorithm selection information
60: observation support information generation unit
60a: nasal cavity observation support information generation unit
60b: nasopharyngeal observation support information generation unit
60c: oropharyngeal observation support information generation unit
60d: hypopharyngeal observation support information generation unit
60e: erroneous insertion observation support information generation unit
60f: observation support information generation unit for out-of-observation-support-target
61: observation support information
70: notification controller
71: guide image
73: observation support information display field
90: upper route
90a: upper route region
91: lower route
91a: lower route region
92: upper route insertion region
93: lower route insertion region
94, 94a, 94b: insertion route guide mark
95: image center guide mark
96: guide frame
100: upper route image
101a, 101b, 101c, 101d, 101e, 101f, 101g, 101h: guide direction display icon
102: guide arrow
103a: endoscope operation support diagram
103b, 103c: lever
110: lower route image
120: anterior nasal image
121: upper route operation display field
122: lower route operation display field
130: nasopharyngeal image
140: oropharyngeal image
141, 151: glottis region
142: epiglottis region
150: hypopharyngeal image
152a, 152b: vocal fold region
160: esophageal image
161: warning display field
170: tracheal image
Af: aryepiglottic fold
Cc: cricoid cartilage
Cot: corniculate tubercle
Cut: cuneiform tubercle
Eg: epiglottis
Ep: nasopharynx
Es: esophagus
Ev: epiglottic vallecula
F: food
Fa: face
Fp: fornix pharyngis
Hp: hypopharynx
It: inferior turbinate
La: larynx
Lw: lateral wall
Mp: oropharynx
Mt: middle turbinate
Nf: nasal floor
Ns: nasal septum
Nw: nasal side wall
Ps: pyriform sinus
Pw: posterior pharyngeal wall
Pwe: posterior wall of nasopharynx
Rg: rima glottidis
Rl: tongue root
Sp: soft palate
Tc: thyroid cartilage
Tr: trachea
To: tongue
Vc: glottis
Vef: vestibular fold
Vof: vocal fold

## Claims

1. An image processing device comprising:
one or more processors (23) configured to:
acquire a medical image (31, 32, 33, 72);
output observation target identification information (41) indicating an observation support target part included in the medical image or indicating that the medical image is out of an observation support target, by inputting the medical image to an observation target identification algorithm;
select, from a plurality of observation support algorithms, one specific observation support algorithm based on the observation target identification information;
output observation support information by inputting the medical image to the specific observation support algorithm; and
perform a control of notifying of the observation support information.

2. The image processing device according to claim 1,
wherein the observation support target part is a nasal cavity, a nasopharynx, an oropharynx, a hypopharynx, a larynx, a trachea, or an esophagus.

3. The image processing device according to claim 1 or 2,
wherein the notification of the observation support information is performed by a guide image for displaying the observation support information and/or a voice for notifying of the observation support information.

4. The image processing device according to claim 3,
wherein the observation support information is endoscope position determination information indicating whether a position of an endoscope is appropriate or inappropriate, endoscope operation information indicating an operation method of the endoscope, subject position information for prompting change or confirmation of a position of a subject, and/or observation support stop information indicating that an observation support is stopped.

5. The image processing device according to claim 4,
wherein the endoscope operation information is a moving direction and/or a moving amount of a distal end part of the endoscope, and
the moving direction is a right direction, a left direction, an upward direction, a downward direction, a backward direction, a forward direction, a right turn, or a left turn.

6. The image processing device according to claim 5,
wherein the one or more processors are configured to perform a control of displaying an endoscope operation support diagram on the guide image as the endoscope operation information.

7. The image processing device according to claim 4,
wherein the one or more processors are configured to perform a control of switching a display of the endoscope operation information based on the endoscope position determination information.

8. The image processing device according to claim 4,
wherein the specific observation support algorithm includes an operation support algorithm, and
the one or more processors are configured to output the endoscope operation information or the observation support stop information, by inputting the medical image to the operation support algorithm.

9. The image processing device according to claim 8,
wherein the one or more processors are configured to input a latest medical image to the operation support algorithm.

10. The image processing device according to claim 9,
wherein the operation support algorithm is a trained model that outputs the endoscope operation information and/or the subject position information.

11. The image processing device according to claim 4,
wherein the specific observation support algorithm includes a position determination algorithm and an operation support algorithm,
the position determination algorithm is a trained model that outputs the endoscope position determination information in response to an input of the medical image, and
the operation support algorithm is a trained model that outputs the endoscope operation information and/or the subject position information in response to an input of the medical image.

12. The image processing device according to claim 11,
wherein the one or more processors are configured to input a latest medical image to each of the position determination algorithm and the operation support algorithm.

13. The image processing device according to claim 1,
wherein the observation target identification algorithm is a trained model that has been trained using a learning image including the medical image in which a nasal cavity, a nasopharynx, an oropharynx, a hypopharynx, a larynx, a trachea, or an esophagus is included in an observation target.

14. The image processing device according to claim 1,
wherein the one or more processors are configured to perform a control of switching between presence and absence of the notification of the observation support information.

15. The image processing device according to claim 3,
wherein the one or more processors are configured to, in a case in which the observation support target part output by the observation target identification algorithm is a nasal cavity:
output insertion region information indicating an upper route insertion region and/or a lower route insertion region, which is a region suitable for insertion of an endoscope, included in the medical image, by inputting the medical image to an operation support algorithm;
calculate insertion route information that is an area, a width, and/or coordinate information of a center position of the upper route insertion region and/or the lower route insertion region based on the insertion region information; and
perform a control of displaying the insertion route information on the guide image as the observation support information.

16. The image processing device according to claim 15,
wherein the one or more processors are configured to perform a control of displaying the upper route insertion region or the lower route insertion region on the guide image by changing a display mode thereof based on the insertion route information.

17. The image processing device according to claim 10,
wherein the one or more processors are configured to, in a case in which the observation support target part output by the observation target identification algorithm is a nasal cavity:
output insertion region information indicating an upper route insertion region and/or a lower route insertion region, which is a region suitable for insertion of the endoscope, included in the medical image, by inputting the medical image to the operation support algorithm;
calculate insertion route information that is an area, a width, and/or coordinate information of a center position of the upper route insertion region and/or the lower route insertion region based on the insertion region information; and
output the endoscope operation information using the insertion route information.

18. The image processing device according to claim 10,
wherein the one or more processors are configured to, in a case in which the observation support target part output by the observation target identification algorithm is a nasopharynx:
output nasopharyngeal position information indicating that the position of the endoscope is in an appropriate position or in an inappropriate direction position that is an inappropriate right position, an inappropriate left position, an inappropriate upper position, an inappropriate lower position, an inappropriate back position, or an inappropriate front position, by inputting the medical image to the operation support algorithm; and
output the endoscope operation information based on the nasopharyngeal position information.

19. The image processing device according to claim 18,
wherein the operation support algorithm is a trained model that has been trained using a learning image including the medical image associated with the nasopharyngeal position information.

20. The image processing device according to claim 11,
wherein the one or more processors are configured to, in a case in which the observation support target part output by the observation target identification algorithm is an oropharynx, and the position determination algorithm to which the medical image is input outputs that the position of the endoscope is inappropriate as the endoscope position determination information:
output oropharyngeal region information indicating a glottis region and/or an epiglottis region included in the medical image, by inputting the medical image to the operation support algorithm;
calculate oropharyngeal region arithmetic information that is an area, a width, and/or coordinate information of a center position of the glottis region and the epiglottis region based on the oropharyngeal region information; and
output the endoscope operation information using the oropharyngeal region arithmetic information.

21. The image processing device according to claim 20,
wherein the position determination algorithm is a trained model that has been trained using a learning image in which the medical image and the endoscope position determination information are associated with each other.

22. The image processing device according to claim 11,
wherein the one or more processors are configured to, in a case in which the observation support target part output by the observation target identification algorithm is a hypopharynx, and the position determination algorithm to which the medical image is input outputs that the position of the endoscope is inappropriate as the endoscope position determination information:
output hypopharyngeal region information indicating a glottis region and/or a vocal fold region included in the medical image, by inputting the medical image to the operation support algorithm;
calculate hypopharyngeal region arithmetic information that is an area, a width, and/or coordinate information of a center position of the glottis region, and that is a length of the vocal fold region based on the hypopharyngeal region information; and
output the endoscope operation information using the hypopharyngeal region arithmetic information.

23. The image processing device according to claim 22,
wherein the position determination algorithm is a trained model that has been trained using a learning image in which the medical image and the endoscope position determination information are associated with each other.

24. The image processing device according to claim 10,
wherein the one or more processors are configured to, in a case in which the observation support target part output by the observation target identification algorithm is an esophagus or a trachea, output the endoscope operation information for providing an instruction to pull out the endoscope by inputting the medical image to the operation support algorithm.

25. The image processing device according to claim 8,
wherein the one or more processors are configured to, in a case in which the observation target identification algorithm outputs that the medical image is out of the observation support target as the observation target identification information, output the observation support stop information, by inputting the medical image to the operation support algorithm.

26. The image processing device according to claim 25,
wherein the observation target identification algorithm is a trained model that has been trained to, in a case in which the medical image including a foreign substance, which is food or saliva, shake, blurriness, or halation is input, output that the medical image is out of the observation support target as the observation target identification information.

27. A method for operating an image processing device, the method comprising:
a step of acquiring a medical image;
a step of outputting observation target identification information indicating an observation support target part included in the medical image or indicating that the medical image is out of an observation support target, by inputting the medical image to an observation target identification algorithm;
a step of selecting, from a plurality of observation support algorithms, one specific observation support algorithm based on the observation target identification information;
a step of outputting observation support information by inputting the medical image to the specific observation support algorithm; and
a step of performing a control of notifying of the observation support information.

28. An endoscope system comprising:
the image processing device according to claim 1;
a light source device that emits illumination light; and
an endoscope that images the medical image.
